(19) Europäisches Patentamt
European Patent Office
Office européen des brevets

(11) Veröffentlichungsnummer: **0 232 893 A2**

(12) # EUROPÄISCHE PATENTANMELDUNG

(21) Anmeldenummer: **87101796.8**

(22) Anmeldetag: **10.02.87**

(51) Int. Cl.⁴: **C 12 P 21/04**, C 07 K 7/06, A 61 K 37/02, A 23 K 1/16

(30) Priorität: **14.02.86 DE 3604678**

(43) Veröffentlichungstag der Anmeldung: **19.08.87 Patentblatt 87/34**

(84) Benannte Vertragsstaaten: **AT BE CH DE ES FR GB GR IT LI LU NL SE**

(71) Anmelder: **HOECHST AKTIENGESELLSCHAFT, Postfach 80 03 20, D-6230 Frankfurt am Main 80 (DE)**

(72) Erfinder: **Franco, Christopher Milton Mathew, Dr., 74A, "The Westminster" N.S. Mankikar Marq, Sion Bombay 400 022 (IN)**
Erfinder: **Mukhopadhyay, Triptikumar, M23/24, Hoechst Quarters Near Jai Shastri Nagar, Mulund (West) Bombay 400 082 (IN)**
Erfinder: **Ganguli, Bimal Naresh, Dr., "Saurayuth" 173 Central Avenue Chembur, Bombay 400 071 (IN)**
Erfinder: **Rupp, Richard Helmut, Dr., 'Niladri' 66 Nepean Sea Road, Bombay 400 006 (IN)**
Erfinder: **Fehlhaber, Hans-Wolfram, Dr., Thomas-Mann-Strasse 5a, D-6270 Idstein/Taunus (DE)**

(54) **Neue Antibiotika vom Streptogramin-Typ, ein mikrobiologisches Verfahren zu ihrer Herstellung und ihre Verwendung als Arzneimittel und Futterzusatzmittel.**

(57) Bei der fermentativen Züchtung des Mikroorganismus Streptomyces species Y-8240155 entstehen neue, als Grividomycin I, II und III bezeichnete Streptogramin-Antibiotika neben dem bekannten Neoviridogrisein als Hauptprodukt. Die Erfindung betrifft Grividomycin I, II und III, die Gewinnung derselben bzw. des die neuen Antibiotika enthaltenden Antibiotikakomplexes auf mikrobiologischem Wege sowie deren Verwendung als Arzneimittel gegen Bakterien, Mycoplasmen und Actinomyceten sowie als wachstumfördernde Zusätze in Tierfuttermitteln.

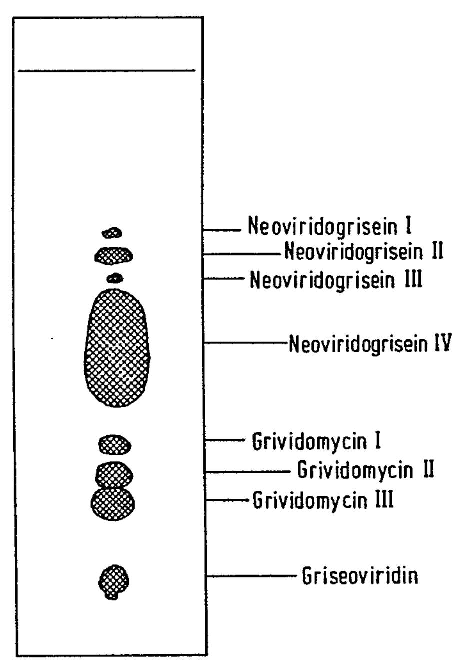


EP 0 232 893 A2

Neue Antibiotika vom Streptogramin-Typ, ein mikrobiologisches Verfahren zu ihrer Herstellung und ihre Verwendung als Arzneimittel und Futterzusatzmittel

Die vorliegende Erfindung betrifft den neuen Mikroorganismus Streptomyces species Y-8240155, ein Verfahren zur Herstellung neuer Antibiotika vom Streptogramin-Typ unter Verwendung dieses Stammes und/oder seiner Mutanten und Varianten sowie die Verwendung der neuen Antibiotika als Arzneimittel und Wirkstoffe für eine Verbesserung der Nährstoffverwertung und Beschleunigung des Wachstums von Nutztieren, insbesondere von Schweinen, Kühen und Geflügel.

Der Mikroorganismus-Stamm Y-8240155 wurde aus einer in Panchgani, Maharashtra, Indien gesammelten Bodenprobe unter Verwendung eines Nährmediums bei pH 6,5 bis 8,5 gewonnen. Varianten und Mutanten des Stammes Y-8240155 sind auf bekannte Weise, z.B. durch Verwendung eines Mutagens wie N-Methyl-N-nitro-N'-nitrosoguanidin erhältlich.

Der Mikroorganismus Streptomyces sp. Y-8240155 gehört zur Ordnung Actinomycetales, der Familie Streptomycetaceae und der Gattung Streptomyces. Mehrere streptograminartige Antibiotika bildende Streptomycesarten sind bekannt und in der Literatur beschrieben (J. Antibiotics 32:575-583, 1979; GB-A 1 575 533). Streptomyces sp. Y-8240155 wird als neuer Stamm angesehen, da er, wie aus der nachfolgenden Beschreibung hervorgeht, sich in einigen seiner morphologischen und physiologischen Eigenschaften sowie seinem Züchtungsverhalten von den bekannten Stämmen unterscheidet. Er wird auch deswegen als neuer Stamm angesehen, weil er, wie aus der nachfolgenden Beschreibung hervorgeht, neue, als Grividomycin I, II und III bezeichnete

0232893

Antibiotika bildet. Der Stamm Streptomyces species Y-8240155 wurde am 15. Januar 1986 bei der "Deutsche Sammlung von Mikroorganismen" in Göttingen unter der Eingangsnummer DSM 3640 hinterlegt.

Durch Züchtung von Streptomyces sp. Y-8040155 (DSM 3640), werden neben dem bekannten Neoviridogrisein IV (US-A 3 023 204) neue Antibiotika erhalten, welche u.a. geeignet sind, in Form von Futterzusätzen das Wachstum von Nutztieren zu beschleunigen.

Die neuen Antibiotika entsprechen der allgemeinen Formel I

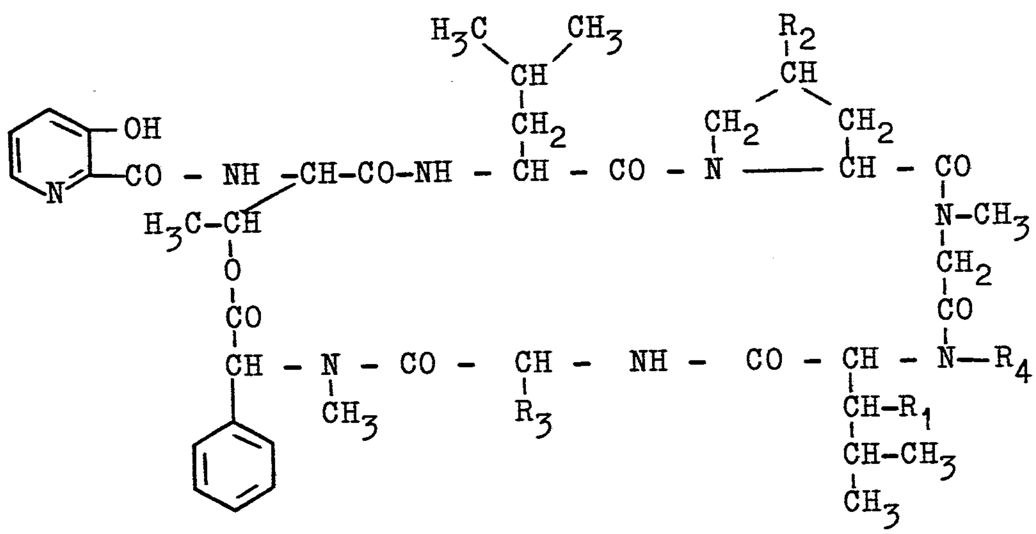

I

worin die Reste $R_1$ bis $R_4$ folgende Bedeutungen haben:

| | $R_1$ | $R_2$ | $R_3$ | $R_4$ |
|---|---|---|---|---|
| Grividomycin I (II) | $CH_3$ | OH | H | $CH_3$ |
| Grividomycin III | $CH_3$ | OH | $CH_3$ | H |

Bei Grividomycin I und II handelt es sich um ein Diastereoisomerenpaar derselben chemischen Formel.

Die neuen erfindungsgemäßen Antibiotika sind gegen zahlreiche Mikroorganismen, wie unter anderem grampositive Bakterien und Mykoplasmen wirksam. Zu solchen Mikroorganismen zählen die Arten Staphylococcus aureus, Streptococcus pyogenes, Diplococcus pneumoniae, Mycoplasma gallinarum und Mycoplasma pulmonis. Die neuen erfindungsgemäßen Antibiotika sind auch bei Stämmen wirksam, die gegen andere Antibiotika resistent geworden sind.

Die neuen erfindungsgemäßen Verbindungen können als Heilmittel oder Futterzusatzmittel in jeder beliebigen bioverfügbaren Form für sich allein oder in Kombination miteinander oder mit den bekannten Neoviridogriseinen I, II, III und/oder mit Neoviridogrisein IV und/oder streptograminartigen Antibiotika der Gruppe A wie Griseoviridin verwendet werden.

Das zur Gewinnung des Mikroorganismus verwendete Nährmedium besteht aus Kohlenstoff- und Stickstoffquellen, anorganischen Nährsalzen und Verfestigungsmitteln. Als Kohlenstoffquellen kommen Glucose, Stärke, Dextrin, Glycerin, Saccharose oder Melasse in Betracht. Geeignete Stickstoffquellen sind Pepton, Hefeextrakt, Kraftbrühe, Malzextrakt, Kasein, Ammoniumsalze, Nitrate oder Aminosäuren. Ein geeignetes Verfestigungsmittel ist beispielsweise Agar. Als anorganische Nährsalze kommen Natrium-, Kalium-, Magnesium-, Kalzium-, Eisen-, Kupfer-, Zink-, Mangan- oder Kobaltsalze oder Phosphate oder Sulfate in Betracht.

Die neuen Grividomycine I, II und III sind Streptograminantibiotika. Letztere gehören zur Gruppe der Depsipeptidverbindungen und sind in zwei Hauptgruppen - A und B - unterteilt (D. Vazquez, Antibiotic III, "Mechanism of action of antimicrobial and antitumor agents" Springer-Verlag Berlin-Heidelberg-New York, 1975, Seite 521 - 534) und werden von Mikroorganismen als Komplex dieser beiden Gruppen gebildet. Antibiotika der Gruppe A und Gruppe B wirken an eng verwandten Stellen auf Ribosomen-50-S-Untereinheiten.

Da die Bindung der Gruppe A-Antibiotika in Gegenwart von Gruppe B-Antibiotika stärker ist, zeigen Antibiotika der Gruppe A einen deutlichen Synergismus mit Antibiotika der Gruppe B in ihrer Wirksamkeit gegen grampositive Bakterien. Zur Gruppe A gehören die folgenden Antibiotika Griseoviridin, Mikamycin A, Ostreogrycin A, Virginiamycin $M_1$, Pristinamycin $II_A$, Synergistin $A_1$; Ostreogrycin G, Virginiamycin $M_2$, Pristinamycin IIB, Dihydroostreogrycin A, Madumycin I und II. Die Gruppe B ist in die Untergruppen Viridogrisein (Neoviridogrisein) und Vernamycin B (Mikamycin B) unterteilt. Die Vertreter der Untergruppe Viridogrisein (Neoviridogrisein) entsprechen der Formel I mit folgenden Bedeutungen für $R_1$, $R_2$ und $R_3$:

| $R_1$ | $R_2$ | $R_3$ | ($R_4 = CH_3$) | Bezeichnung |
|---|---|---|---|---|
| $CH_3$ | OH | $CH_3$ | | Viridogrisein I<br>Etamycin, F 1370 A<br>Neoviridogrisein IV |
| H | OH | $CH_3$ | | Viridogrisein II |
| $CH_3$ | H | $C_2H_5$ | | Neoviridogrisein I |
| $CH_3$ | H | $CH_3$ | | Neoviridogrisein II |
| $CH_3$ | OH | $C_2H_5$ | | Neoviridogrisein III |

Die Neoviridogriseine (NVG) I, II und III ein Verfahren zu ihrer Herstellung unter Verwendung des Stammes Streptomyces species P 8648 (FERM-P 3562) sowie ihre Verwendung als Arzneimittel oder Futterzusatz, sind bereits aus der DE-A 2 725 163 bekannt. Neoviridogrisein IV ist aus US-A 3,023,204 als Antibiotikum mit Wirkung gegen Actinomyceten, Bakterien, Rickettsien und Viren, insbesondere zur Behandlung von Mastitis bekannt.

Die Untergruppe Vernamycin B (Mikamycin B) ist in Formel II

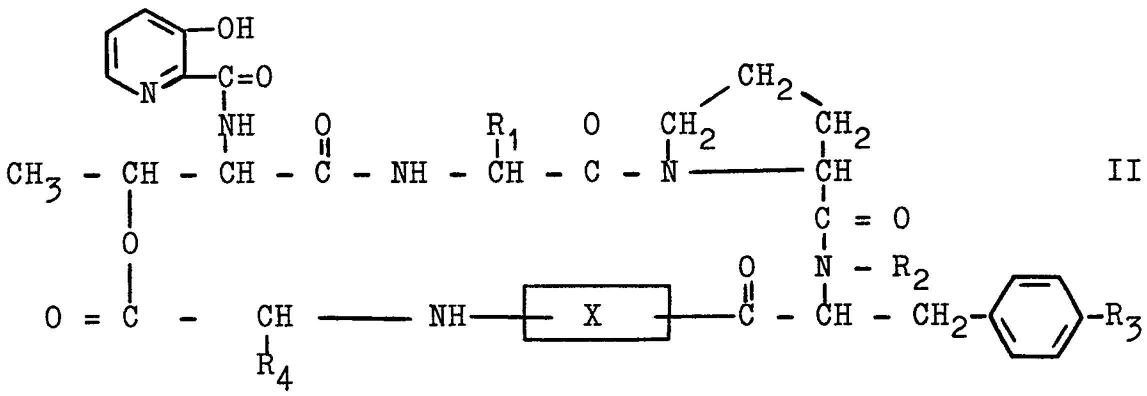

II

gezeigt, worin $R_1$, $R_2$, $R_3$, $R_4$ und X die folgenden Bedeutungen haben:

| $R_1$ | $R_2$ | $R_3$ | X | $R_4$ | Bezeichnung |
|---|---|---|---|---|---|
| $C_2H_5$ | $CH_3$ | $N(CH_3)_2$ | 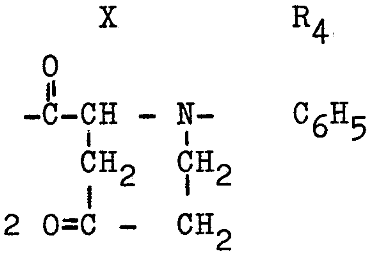 (4-Oxopipecolinsäure) | $C_6H_5$ | Ostreogrycin B<br>Mikamycin B<br>Streptogramin B<br>Synergistin B-1<br>Vernamycin $B_2$<br>Pristinamycin $I_A$ |
| $CH_3$ | $CH_3$ | $N(CH_2)_2$ | (4-Oxopipecolinsäure) | $C_6H_5$ | Ostreogrycin $B_1$<br>Pristinamycin $1_c$<br>Vernamycin $B_1$ |
| $C_2H_5$ | $CH_3$ | $NHCH_3$ | (4-Oxopipecolinsäure) | $C_6H_5$ | Ostreogrycin $B_2$<br>Pristinamycin $1_B$<br>Vernamycin B |
| $C_2H_5$ | $CH_3$ | $N(CH_3)_2$ | 3-Hydroxy-4-oxopipecolinsäure | $C_6H_5$ | Ostreogrycin $B_3$ |
| $C_2H_5$ | $CH_3$ | H | Prolin | $C_6H_5$ | Patricin A |
| $C_2H_5$ | $CH_3$ | H | Pipecolinsäure | $C_6H_5$ | Patricin B |
| $CH_3$ | $CH_3$ | $NHCH_3$ | 4-Oxopipecolinsäure | $C_6H_5$ | Vernamycin B |

| | | | | | |
|---|---|---|---|---|---|
| $C_2H_5$ | $CH_3$ | $N(CH_3)_2$ | Asparagin-säure | $C_6H_5$ | Vernamycin C |
| $C_2H_5$ | $CH_3$ | H | 4-Oxopipe-colinsäure | $C_6H_5$ | Virginiamycin S |
| $C_2H_5$ | H | H | 4-Hydroxy-pipecolin-säure | $C_6H_5$ | " $S_2$ |
| $C_2H_5$ | $CH_3$ | H | 3-Hydroxy-4-oxopipe-colinsäure | $C_6H_5$ | " $S_3$ |
| $CH_3$ | $CH_3$ | H | 4-Oxopipe-colinsäure | $C_6H_5$ | " $S_4$ |
| $C_2H_5$ | $CH_3$ | H | 4-Hydroxy-pipecolin-säure | $CH_3$ | " $S_5$ |

Gemäß ihren physikalisch-chemischen und spektroskopischen Daten gehören die obengenannten von Streptomyces species Y-8240155 gebildeten neuen Antibiotika zur Viridogrisein-Untergruppe der Streptogramingruppe B, unterscheiden sich aber von den bekannten Neoviridogriseinen I, II, III und IV in ihrem Dünnschichtchromatogramm (siehe Figur 1 der beiliegenden Zeichnungen), Hochleistungsflüssigkeitschromatogramm, ihren physikalisch-chemischen und ihren spektroskopischen Eigenschaften. Das zur Dünnschichtchromatographie (DC) verwendete Lösungsmittelsystem war 3%iges Methanol in Chloroform, und der Nachweis erfolgte bei 366 nm/254 nm.

Der erfindungsgemäße Mikroorganismus bildet längliche farblose Luftmyzelien aus verzweigten Substratmyzelien. Auf den Luftmyzelien werden spiralförmige Sporenketten gebildet. Es wird weder Wirbel- noch Askosporenbildung beobachtet. Die Eigenschaften der Mikroorganismen bei der Kultur auf verschiedene Agarmedien werden wie folgt beschrieben:

1. Hefeextrakt-Malzextraktagar

Wachstum: Gut

Luftmyzel: Reichlich, weisslich, samtig-baumwollartig, gelegentlich Wassertröpfchen auf der Oberfläche.

Rückseite: Hellbraun

Lösliches Pigment: Keines

2. Hafermehlagar

Wachstum: Gut

Luftmyzel: Gut, weisslich, samtig

Rückseite: Schwach gelb bis hellbraun

Lösliches Pigment: Keines

3. Anorganische Salze-Stärkeagar

Wachstum: Gut

Luftmyzel: Gut, weiss

Rückseite: Hellbraun

Lösliches Pigment: Keines

Stärkehydrolyse: Positiv

4. Glycerin-Asparaginagar

Wachstum: Mäßig

Luftmyzel: Schwach oder keines. Wenn vorliegend, dann weiss.

Rückseite: Hellgelb

Lösliches Pigment: Keines

5. Tyrosinagar

Wachstum: Gut

Luftmyzel: Mäßig, weiss

Rückseite: Braun

Lösliches Pigment: Braun, Melaninpigment gebildet

6. Saccharose-Nitratagar

Wachstum: Mäßig

Luftmyzel: Keines
Rückseite: Schwach gelb
Lösliches Pigment: Keines

7. Glucose-Asparaginagar
Wachstum: Mäßig
Luftmyzel: Schwach, weiss
Rückseite: Schwach gelb
Lösliches Pigment: Keines

8. Nähragar
Wachstum: Mäßig
Luftmyzel: Keines
Rückseite: Hellbraun
Lösliches Pigment: Schwach braun

9. Pepton-Hefeextrakt-Eisenagar
Wachstum: Mäßig
Luftmyzel: Keines
Rückseite: Braun
Lösliches Pigment: Braun

Der optimale Wachstumstemperaturbereich für den erfindungsgemäßen Mikroorganismus liegt bei 25 bis 35°C. Der Stamm verflüssigt Gelatine in sehr geringem Umfang in Glucose-Pepton-Gelatinemedium, hydrolysiert Stärke in anorganischem Salz-Stärkeagar und peptonisiert Magermilch.

Die Bildung von Melaninpigment wird in Tyrosinagar, Pepton-/Hefeextrakt-Eisenagar und Trypton-Hefeextraktbouillon beobachtet.

Das Kohlenstoffquellen-Assimilationsschema dieses Mikroorganismus ist wie folgt (in Pridham-Gottlieb-Medium):

Positiv D-Xylose, D-Glucose, L-Arabinose, I-Inosit, Rhamnose, Raffinose, D-Mannit

Schwach positiv: Saccharose, D-Fruktose

Im Hinblick auf die Bildung von bekannten Streptogramin-antibiotika wie Mikamycin A und B, Virginiamycine, Ostreogrycine, Etamycin, Vernamycin, Viridogrisein, Griseoviridin und Pristinamycine, sollten die folgenden bekannten Mikroorganismen mit Streptomyces species Y-8240155 verglichen werden:

Streptomyces sp. P8648
Streptomyces griseus NRL 2426
Streptomyces griseoviridus NRL 2427
Streptomyces sp., Etamycinbildner
Streptomyces conganensis
Streptomyces ostreogrisius
Streptomyces mitakensis
Streptomyces loidensis
Streptomyces griseoroseus
Streptomyces lavendulae

Die veröffentlichten Daten über die Züchtungs- und physiologischen Eigenschaften des genannten Mikroorganismus zeigen klare Unterschiede zwischen dem erfindungsgemäßen Mikroorganismus und den obengenannten bekannten Mikroorganismen. Beispielsweise unterscheidet sich Streptomyces griseus NRRL 2426 dadurch, daß er zur gelben Reihe, Section Rectiflexibiles, gehört und keine dunklen Pigmente bildet, wogegen der erfindungsgemäße Mikroorganismus zur weißen Reihe, Section Spirales, gehört und dunkles Pigment bildet. Streptomyces griseovirides NRRl 2427 läßt sich von dem erfindungsgemäßen Mikroorganismus anhand der Züchtungs-Eigenschaften auf Hefeextrakt-Malzextraktagar, Hafermehlagar, Glycerin, Asparaginagar und der Bildung von dunklem Pigment unterscheiden. Streptomyces sp. P8648 zeigt klare Unterschiede von dem erfindungsgemäßen Mikroorganismus in seinem Kohlenstoffquellen-Assimilationsschema und den Züchtungs-Eigenschaften auf Czapek-Agar, anorganischen Salzen, Stärkeagar und Hafermehlagar. Darüberhinaus bildet der Stamm P8648 kein dunkles Pigment, wogegen der Stamm Y-8240155 dieses bildet. Die Ergebnisse eines taxonomischen Vergleichs zwischen den beiden Stämmen sind in der nachfolgenden Tabelle I zusammengefaßt:

Tabelle I

| | Streptomyces sp. P8648 | Streptomyces sp. Y-8240155 |
|---|---|---|
| Farbe des Luftmyzels | Weisses bis grauweisses Luftmyzel wird auf den meisten ISP-Medien nur schlecht gegebildet. Weisses Luftmyzel wird auf Hefeextrakt-Malzextraktagar reichlich gebildet. | Weises Luftmyzel wird auf den meisten ISP-Medien mäßig bis reichlich gebildet. |
| Farbe des Substratmyzels | Schwach gelb bzw. hellgelb bis graubraun auf den meisten ISP-Medien. | Schwach gelb bis hellbraun bis braun auf den meisten ISP-Medien. |
| Lösliches Pigment | Kein dunkles Pigment gebildet. Gewöhnlich kein weiteres Pigment beobachtbar, aber in seltenen Fällen schlecht gebildetes gelbes Pigment. | Dunkles Pigment gebildet. In seltenen Fällen braunes Pigment schwach gebildet. |
| Verwertung der Kohlenstoffquellen | L-Arabinose -<br>I-Inosit -<br>Raffinose - | L-Arabinose +<br>I-Inosit +<br>Raffinose + |

Wie aus den vorstehenden Daten ersichtlich, wurden zwischen Str. sp. P8648 und den erfindungsgemäßen Streptomyzeten klare Unterschiede in den physiologischen und Züchtungs-Eigenschaften und dem Kohlenstoffquellen-Verwertungsschema bestätigt. Darüberhinaus bildet der erfindungsgemäße Mikroorganismus bei seiner Fermentation in der Hauptsache Neoviridogrisein IV (Viridogrisein I), daneben Grividomyin I, II und III und Griseoviridin, während Streptomyces sp. P8648 nur Neoviridogriseine I, II, III, Viridogrisein (Neoviridogrisein IV) und Griseoviridin, nicht aber Grividomycin I, II und III bildet. Außerdem ist der Anteil an Griseoviridin im Fermentationsprodukt des neuen Mikroorganismus nur gering ($\leqq$ 2 %), während die bekannten Streptomyces sp. P8646, Streptomyces griseus NRRL 2426 und S. griseoviridis NRRL 2427 durchweg beträchtliche Mengen (>20 %) Griseoviridin bilden. Aufgrund der obigen Ergebnisse wird der erfindungsgemäße Mikroorganismus als eine neue Streptomycesart angesehen.

Gegenstand der vorliegenden Erfindung ist auch ein Verfahren zur Herstellung von zur Klasse der Streptogramin-antibiotika gehörenden Verbindungen, das dadurch gekennzeichnet ist, daß man Streptomyces sp. Y-8240155 (DSM 3640) oder seine natürlichen und künstlichen Varianten und Mutanten durch Fermentation bei einem pH zwischen 6,5 und 8,5 und einer Temperatur zwischen 20 und 40°C unter aeroben Bedingungen in einem Kohlenstoff- und Stickstoffquellen, anorganische Nährsalze und Spurenelemente enthaltenden Nährmedium züchtet und die Verbindungen in herkömmlicher Weise aus den Kulturbrühen isoliert.

Die im Nährmedium zur Herstellung der neuen Antibiotika verwendeten Kohlenstoffquellen können Glucose, Stärke, Dextrin, Glycerin, Saccharose, Melasse und Oel sein. Geeignete, in dem Nährmedium zur Herstellung der neuen Antibiotika verwendete Stickstoffquellen sind Sojabohnenmehl, Hefeextrakt, Kraftbrühe, Malzextrakt, Maisquellwasser,

Peptone oder Kasein. Geeignete anorganische Nährsalze sind Natriumchlorid, Magnesiumsulfat, Ammoniumsulfat und Kalziumcarbonat. Als Spurenelemente kommen Eisen, Mangan, Kupfer, Zink und Kobalt in Betracht.

Streptomyces sp. Y-8240155 (DSM 3640) wird vorzugsweise bei 25-30°C und pH 6,5-7,0 kultiviert. Die Fermentation wird vorteilhaft nach 40 bis 50 Stunden abgebrochen, wonach man Höchstausbeuten der Verbindungen erhält. Bei der Fermentation handelt es sich vorzugsweise um eine Submersfermentation.

Der Fermantationsverlauf und die Bildung der Streptograminverbindungen lassen sich anhand der antibakteriellen Wirksamkeit der Kulturflüssigkeit und des Myzels gegen Staphylococcus aureus 209 P kontrollieren.

Bei der fermentativen Züchtung von Streptomyces sp. Y-8240155 DSM 3640 wird in der Hauptsache Neoviridogrisein IV, daneben Grividomycin I, II und III und Neoviridogrisein II, außerdem gegebenenfalls Griseoviridin und Neoviridogrisein I und III gebildet. Die Anteile der einzelnen Komponenten können je nach Zusammensetzung des Kulturmediums innerhalb weiter Grenzen schwanken. In der Regel enthält das isolierte Fermentationsprodukt ca. 90 % oder mehr NVG IV, während die übrigen Bestandteile zusammen nicht mehr als 10 % ausmachen. Die Fermentationsprodukte können bei pH 4,5 bis 7,5 - vorzugsweise bei pH 6,5 - aus dem Kulturfiltrat als Gemisch unter Verwendung herkömmlicher Methoden wie Extraktion mit nicht mit Wasser mischbaren Lösungsmitteln wie zum Beispiel Chloroform, Methylenchlorid, n-Butanol, Essigester oder Butylacetat (vorzugsweise Essigester) isoliert werden.

Ein anderes Verfahren für die Gewinnung der antibiotischen Verbindungen aus der Kulturbrühe beruht auf der Adsorption. Das Kulturfiltrat oder eine andere Lösung des Antibiotikakomplexes wird dabei einer Säulenchromatographie oder dergleichen unterworfen, wobei geeignete Adsorbentien wie Aktivkohle, Diaion$^{(R)}$HP 20, Amberlite$^{(R)}$XAD, Sephadex$^{(R)}$

LH-20 oder deren Äquivalente verwendet werden. Der Antibiotikakomplex kann mittels geeigneter mobiler Phasen wie Aceton, oder Methanol (allein oder in Kombination miteinander oder mit Wasser) vom Adsorbens eluiert werden. Die Eluate werden zur Trockne eingedampft und nach den obigen Verfahren weiter gereinigt. In einem geeigneten Stadium des Reinigungsprozesses kann der Antibiotikakomplex durch Ausfällung mit Hexan oder Petroläther entfettet werden.

Die Grividomycine I, II und III können unter Verwendung chromatographischer Routinemethoden von den Neoviridogriseinen I, II, III, IV und Griseoviridin abgetrennt werden. Bei solchen Methoden kann es sich um Schwerkrafts-, Mitteldruck-Kieselgel-Säulenchromatographie, Sephadex$^{(R)}$-LH-20-Chromatographie, Tröpfchen-Gegenstromchromatograhie mit einem geeigneten Lösungsmittelsystem, präparative Hochleistungsflüssigkeitschromatographie auf LiChrosorb$^{(R)}$-RP-18 oder Dünnschichtchromatographie handeln.

Aus dem Mycel kann der antibiotisch wirksame Komplex durch Extraktion des Myzelkuchens mit einem organischen Lösungsmittel, vorzugsweise Aceton gewonnen werden. Der Acetonextrakt wird konzentriert, die wäßrige Schicht auf pH 4,5 bis 7,5 - vorzugsweise pH 6,5 - eingestellt und mit nicht mit Wasser mischbaren organischen Lösungsmitteln wie Chloroform, Methylenchlorid, n-Butanol, Essigester oder Butylacetat, wobei Essigester als Lösungsmittel bevorzugt ist, extrahiert. Die Essigesterextrakte des Myzels und das Kulturfiltrat werden vereinigt oder getrennt aufgearbeitet. Wenn nötig, kann das Mycel mit Wasser gewaschen und das antibiotikahaltige Waschwasser mit den Kulturfiltraten vereinigt oder separat aufgearbeitet werden.

Die Grividomycine I, II und III sind farblose amorphe Pulver und in Methanol, Aethanol, n-Propanol, iso-Propanol, n-Butanol, Essigester, n-Butylacetat, Aceton, Aethylmethylketon, Methylisobutylketon, Benzol, Toluol, Methylenchlorid, Chloroform, Dimethylformamid und Dimethylsulfoxyd löslich, Hexan, Petroläther (40-60), Diäthyläther

und Wasser schwer- oder unlöslich. Sie haben folgende Schmelzpunkte:

| | |
|---|---|
| Grividomycin I: | 141 - 145°C |
| Grividomycin II: | 153 - 157°C |
| Grividomycin III: | 197 - 198°C |

Das Molekulargewicht der drei Grividomycine beträgt 864.

Die spektroskopischen Daten sind in den beigefügten Zeichnungen wie folgt aufgeführt:

Fig. 2 IR-Spektrum von Grividomycin III (in KBr)
Fig. 3 Massenspektrum von Grividomycin III
Fig. 4 IR-Spektrum von Grividomycin II (in KBr)
Fig. 5 Massenspektrum von Grividomycin II
Fig. 6 IR-Spektrum von Grividomycin I (in KBr)

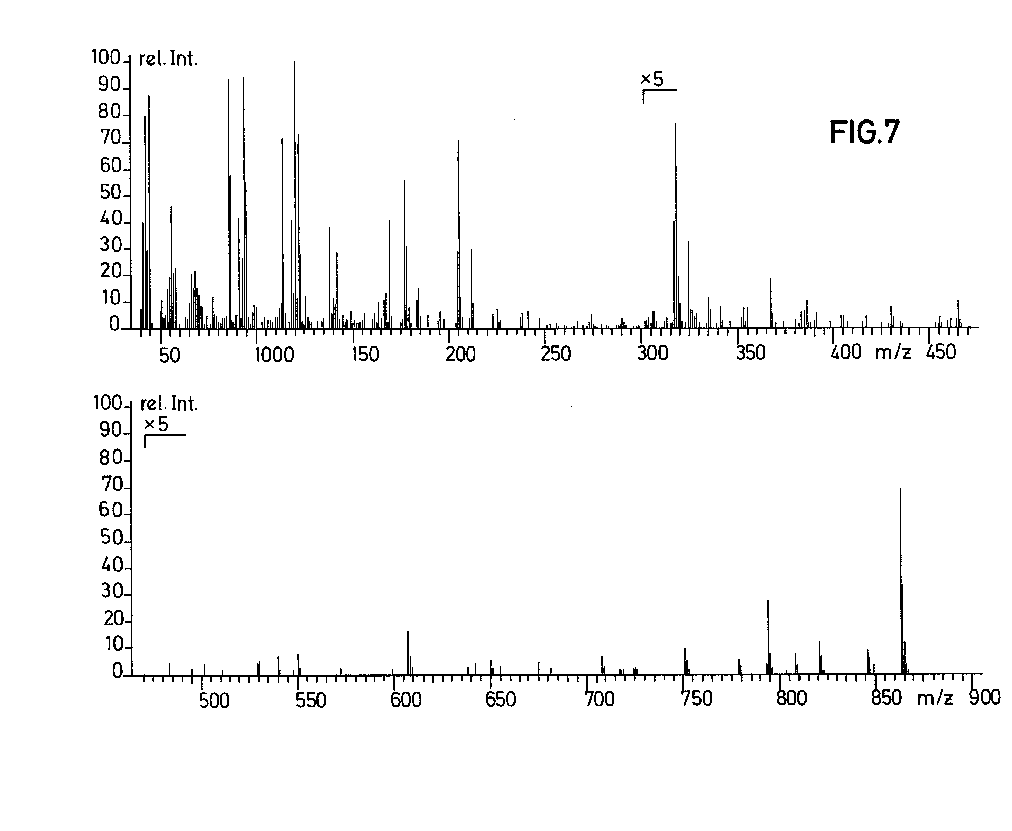

Fig. 7 Massenspektrum von Grividomycin I

Im Dünnschichtchromatogramm (Kieselgel-Fertigplatte) haben die Grividomycine, I, II und III die folgenden $R_f$-Werte:

| | $R_f$ Methanol in Chloroform | | $R_f$ Methanol in Essigester | |
|---|---|---|---|---|
| | 3 % | 7,5 % | 5 % | 10 % |
| Grividomycin III | 0,18 | 0,41 | 0,15 | 0,25 |
| Grividomycin II | 0,23 | 0,48 | 0,21 | 0,34 |
| Grividomycin I | 0,29 | 0,59 | 0,28 | 0,40 |

Bei der analytischen Hochleistungsflüssigkeitschromatographie (HPLC) zeigen die Grividomycine I, II und III die folgenden Retentionszeiten:

Säulenfüllung: LiChrosorb(R)-RP-18/7µ Teilchengröße [0,4x(25+3)cm]

| | |
|---|---|
| Durchfluß: | 1 ml/min |
| Nachweis: | bei 305 nm |
| Lösungsmittel: | Acetonitril / 25 mM Ammoniumacetat / Essigsäure (150:100:3) |

| | Retentionszeit |
|---|---|
| Grividomycin III | 7,0 min |
| Grividomycin II | 6,8 min |
| Grividomycin I | (Die Mischung konnte im obengenannten Lösungsmittelsystem nicht aufgetrennt werden.) |

Antibakterielle Wirksamkeit

Die Grividomycine I, II und III sind gegen Bakterien, Mykoplasmen und Actinomyceten in Labortests antimikrobiell wirksam. Sie zeigen eine in-vitro-Wirksamkeit gegen die empfindlichen und resistenten Stämme von Staphylococcus aureus sowie gegen Stämme von Streptococcus pyogenes, Diplococcus pneumoniae, Sarcina lutea, Bacillus subtilis, Mycoplasma gallinarum und Mycoplasma pulmonis. Die minimale Hemmkonzentration der neuen Antibiotika wurde an verschiedenen Mikroorganismen bestimmt. Die Ergebnisse sind in der nachfolgenden Tabelle im Vergleich zu NVG II und NVG IV gezeigt.

Im Dünnschichtchromatogramm (Kieselgel-Fertigplatte) haben die Grividomycine, I, II und III die folgenden $R_f$-Werte:

| | $R_f$ Methanol in Chloroform | | $R_f$ Methanol in Essigester | |
|---|---|---|---|---|
| | 3 % | 7,5 % | 5 % | 10 % |
| Grividomycin III | 0,18 | 0,41 | 0,15 | 0,25 |
| Grividomycin II | 0,23 | 0,48 | 0,21 | 0,34 |
| Grividomycin I | 0,29 | 0,59 | 0,28 | 0,40 |

Bei der analytischen Hochleistungsflüssigkeitschromatographie (HPLC) zeigen die Grividomycine I, II und III die folgenden Retentionszeiten:

Säulenfüllung: LiChrosorb(R)-RP-18/7μ Teilchengröße [0,4x(25+3)cm]

| | |
|---|---|
| Durchfluß: | 1 ml/min |
| Nachweis: | bei 305 nm |
| Lösungsmittel: | Acetonitril / 25 mM Ammoniumacetat / Essigsäure (150:100:3) |

| | Retentionszeit |
|---|---|
| Grividomycin III | 7,0 min |
| Grividomycin II } Grividomycin I } | 6,8 min (Die Mischung konnte im obengenannten Lösungsmittelsystem nicht aufgetrennt werden.) |

Antibakterielle Wirksamkeit

Die Grividomycine I, II und III sind gegen Bakterien, Mykoplasmen und Actinomyceten in Labortests antimikrobiell wirksam. Sie zeigen eine in-vitro-Wirksamkeit gegen die empfindlichen und resistenten Stämme von Staphylococcus aureus sowie gegen Stämme von Streptococcus pyogenes, Diplococcus pneumoniae, Sarcina lutea, Bacillus subtilis, Mycoplasma gallinarum und Mycoplasma pulmonis. Die minimale Hemmkonzentration der neuen Antibiotika wurde an verschiedenen Mikroorganismen bestimmt. Die Ergebnisse sind in der nachfolgenden Tabelle im Vergleich zu NVG II und NVG IV gezeigt.

Tabelle II

| Testorganismus | MHK-Werte [µg/ml] | | | | |
|---|---|---|---|---|---|
| | NVG II | NVG IV | Grivido I | Grivido II | Grivido III |
| S. aureus 209 P | 0.4 | 1.6 | 6.4 | 0.4 | 6.4 |
| S. aureus 3066 Meth.$^R$ | 0.4 | 1.6 | 25 | 3.2 | 3.2 |
| S. aureus R 85 Tet$^R$ | 0.4 | 1.6 | 25 | 6.4 | 6.4 |
| S. aureus R 85/M Em$^R$ | 1.6 | 6.4 | >50 | 50.0 | 50.0 |
| S. aureus 712 Meth$^R$ | 0.2 | 1.6 | 25 | 12.5 | 25.0 |
| S. aureus 789 Meth$^R$ | 0.8 | 3.2 | 25 | 12.5 | 50.0 |
| Str. faecalis 02DODU1 Mac$^R$ | 12.5 | 12.5 | >50 | 50.0 | >50.0 |
| Str. faecalis UD8b | 6.4 | 25 | >50 | >50.0 | 50.0 |
| Str. faecalis Eder Mac$^R$ | 25 | 25 | >50 | 50.0 | >50.0 |
| Micrococcus luteus | 0.4 | 1.6 | 12.5 | 0.4 | 6.2 |
| Sarcina lutea | 0.4 | 1.6 | 12.5 | 3.2 | 12.5 |
| Bacillus subtilis | 0.2 | 0.4 | 3.2 | 1.6 | 12.5 |
| E. coli 9632 | >50 | >50 | >50 | >50.0 | >50.0 |
| E. coli Ess 2231 | >50 | >50 | >50 | >50.0 | >50.0 |
| Alkaligenes faecalis | >50 | >50 | >50 | >50.0 | >50.0 |
| Ps. aeruginosa M 35 | >50 | >50 | >50 | >50.0 | >50.0 |

Meth$^R$ = methicillin resistent; Tet$^R$ = tetracyclin resistent;
Em$^R$ = erythromycin resistent; Mac$^R$ = macrolid-antibiotika-resistent.

Wachstumsfördernde Wirkung

Der erfindungsgemäß im wesentlichen erhaltene Antibiotikakomplex - bestehend aus Neoviridogrisein IV als Hauptbestandteil nebst den neuen Grividomycinen I, II und III - zeigt eine ausgeprägte wachstumsfördernde Wirkung in Fütterungsversuchen bei Tieren, z.B. bei Mastgeflügel, Schweinen, Mastrindern, Schafen und Kaninchen. Dies gilt auch für die einzelnen Komponenten, insbesondere für das Neoviridogrisein IV, für das ein wachstumsfördernder Effekt bisher nicht beschrieben war. Auch die Legeleistung von Geflügel, insbesondere das Eigewicht und die Milchleistung von Milchkühen, -schafen und -ziegen wird durch den Antibiotikakomplex oder seine einzelnen Komponenten positiv beeinflußt.

Zur Erzielung des Effekts verwendet man Konzentrationen von 2 - 100 ppm, vorzugsweise 2 - 50 ppm, insbesondere 5 - 20 ppm, bezogen auf Alleinfutter. Entsprechend höher sind die Konzentrationen, wenn die Antibiotika als Zusatz zu Beifutter verabreicht werden.

Die Erfindung wird durch die nachfolgenden Beispiele veranschaulicht:

Beispiel I

Gewinnung von Streptomyces Y-8240155 aus einer Bodenprobe

(a) Herstellung eines Nährmediums

| | | | |
|---|---|---|---|
| Medium 1: | Glucose | 1,0 | g |
| | Glycerin | 1,0 | g |
| | L-Arginin | 0,3 | g |
| | $K_2HPO_4$ | 0,3 | g |
| | $MgSO_4 \cdot 7H_2O$ | 0,2 | g |
| | NaCl | 0,3 | g |
| | Hefeextrakt | 2,0 | g |
| | $Fe_2(SO_4)_3$ | 10 | mg |
| | $CuSO_4 \cdot 5H_2O$ | 1 | mg |
| | $ZnSO_4 \cdot 7H_2O$ | 1 | mg |
| | $MnSO_4 \cdot 7H_2O$ | 1 | mg |
| | Agar | 15,0 | g |
| | Destilliertes Wasser | 1 | Liter |
| | pH | 6,5 | |

| | | | |
|---|---|---|---|
| Medium 2: | Glucose | 2,0 | g |
| | L-Asparagin | 1,0 | g |
| | $K_2HPO_4$ | 0,5 | g |
| | $MgSO_4 \cdot 7H_2O$ | 0,5 | g |
| | Bodenextrakt | 200 | ml |
| | Agar | 15,0 | g |
| | Destilliertes Wasser | 800 | ml |
| | pH | 8,0 | |

| | | |
|---|---|---|
| Medium 3: | Stärke | 10,0 g |
| | Kasein | 0,3 g |
| | $KNO_3$ | 2,0 g |
| | NaCl | 2,0 g |
| | $K_2HPO_4$ | 2,0 g |
| | $MgSO_4 \cdot 7H_2O$ | 0,05 g |
| | $CaCO_3$ | 0,02 g |
| | $FeSO_4$ | 0,01 g |
| | Agar | 15,0 g |
| | Destilliertes Wasser | 1 Liter |
| | pH | 7,2-7,5 |

Die Medien wurden bei 121°C eine halbe Stunde lang sterilisiert.

(b) Herstellung der Bodensuspension

1 Gramm Erde wurde in destilliertem Wasser suspendiert und gut umgeschüttelt. Man ließ die Erde sich absetzen und verwendete den Überstand zur Beimpfung eines jeden der obengenannten Isolierungsmedien.

(c) Beimpfung des Isolierungsmediums

Ein ml der Erdsuspension wurde auf 50 ml eines beliebigen der obengenannten Isolierungsnährmedien enthaltende Petri-Schalen überimpft.

(d) Isolierung von Streptomyces sp. Y-8240155

Die beimpfte Petri-Schale wurde zwei Wochen lang bei 30°C inkubiert und Streptomyces sp. Y-8240155 wurde aus den wachsenden Mikroorganismen gewonnen.

Beispiel II
Züchtung von Streptomyces sp. Y-8240155 (DSM 3640) für die fermentative Herstellung des Antibiotikakomplexes

Streptomyces sp. Y-8240155 wurde auf Hefe-Malzagar mit folgender Zusammensetzung gezüchtet:

| | |
|---|---|
| Malzextrakt | 10,0 g |
| Hefeextrakt | 4,0 g |
| Glucose | 4,0 g |
| Agar | 15,0 g |
| Destilliertes Wasser | 1 Liter |
| pH | 7,0 |

Das Medium wurde in Reagenzgläser verteilt und 30 Minuten lang bei 121°C sterilisiert. Die Gläser wurden in Schräglage zur Herstellung von Schrägagars gekühlt. Die Schrägagars wurden mit der Kultur beimpft und 10 bis 15 Tage lang bei 25°C inkubiert, wonach gutes Wachstum und Sporenbildung beobachtet wurden. Eine Suspension der Sporen eines Schrägagars in destilliertem Wasser wurde zur Beimpfung von fünf jeweils 100 ml des Impfkulturmediums enthaltenden 500 ml-Erlenmeyerkolben oder einer 1 l desselben Impfkulturmediums enthaltenden 5 l-Saugflasche verwendet.

<u>Zusammensetzung des Impfkulturmediums</u>

| | |
|---|---|
| Glucose | 15,0 g |
| Sojabohnenmehl | 15,0 g |
| Maisquellwasser | 5,0 g |
| $CaCO_3$ | 2,0 g |
| NaCl | 5,0 g |
| Destilliertes Wasser | 1 Liter |
| pH | 7,0 |

Jeweils 100 ml-Portionen des obigen Mediums wurden in 500 ml-Erlenmeyerkolben bzw. 1 Liter-Portionen in 5 l Saugflaschen verteilt und 30 Minuten lang bei 121°C sterilisiert. Die Kolben/Flaschen wurden abgekühlt, mit Sporensuspension beimpft und bei 240 U.p.M. 72 Stunden lang bei 30°C auf einer Rotationsschüttelmaschine mit einem Ausschlag von 3,8 cm (1 1/2 Zoll) geschüttelt. Das resultierende Wachstum wurde zur Beimpfung von zwei jeweils 10 l eines 8-10 Vol.-%igen Impfkulturmediums enthaltenden 15 l-Glasfermentern zur Herstellung der zweiten Stufe der Impfkultur

verwendet. Die Fermentation wurde bei 27°C (± 1°C) unter Rühren bei 160-180 U.p.M. und einer Belüftungsmenge von 6-7 lpm 24 Stunden lang durchgeführt. Die erhaltene, gut gewachsene zweite Stufe der Impfkultur wurde zur Beimpfung des Herstellungsmediums verwendet.

Zusammensetzung des Herstellungsmediums

| | |
|---|---|
| Glucose | 15,0 g |
| Lösliche Stärke | 20,0 g |
| Sojamehl | 3,0 g |
| Pepton | 3,0 g |
| $CaCO_3$ | 2,0 g |
| NaCl | 2,0 g |
| Maisquellwasser | 2,0 g |
| $(NH_4)_2SO_4$ | 0,5 g |
| Destilliertes Wasser | 1 Liter |
| pH | 7,0 |

Der Fermenterinhalt wurde mit 0,025 % Desmophen(R) als Entschäumungsmittel versetzt.

280 l des obigen Mediums wurden in einem 390 l-Fermenter vorgelegt. Das Medium wurde 28 Minuten lang bei 121°C durch indirekten und direkten Dampf sterilisiert. Der Fermenter wurde gekühlt und mit der zweiten Stufe der Impfkultur (7 % v/v) beimpft. Die Fermentation wurde bei 27°C (± 1°C) unter Rühren bei 100-120 U.p.M. 40-44 Stunden lang durchgeführt. Die Belüftung erfolgte mit einer Menge von 170 Litern pro Minute. Am Ende der Fermentation nach 40 bis 44 Stunden betrug die Antibiotikumkonzentration 40-60 mg pro Liter, und der pH der Kulturflüssigkeit lag im Bereich 6,5-7,0. Der Mycelanteil betrug 5-6 Vol-%.

Die abgeerntete, den Antibiotikakomplex enthaltende Kulturbouillon wurde zur Trennung des Myzels von der Kulturflüssigkeit zentrifugiert. Diese wurden dann gemäß dem Ablaufschema in dem nachfolgenden Diagramm aufgearbeitet:

## Diagramm I

**Kulturfiltrat**

1. pH auf 6,5 eingestellt
2. Extraktion mit Essigester
3. Lösungsmitteldestillation unter vermindertem Druck

Konzentrierter Extrakt

**Myzel**

1. Acetonextraktion
2. Konzentration
3. Verdünnung des Konzentrats mit Wasser
4. pH eingestellt auf 6,5
5. Extraktion mit Essigester
6. Lösungsmitteldestillation unter vermindertem Druck

Konzentrierter Extrakt

Roher Antibiotikakomplex

Flashsäulenchromatographie (Kieselgel)

1-2% Methanol in Chloroform

Neoviridogrisein I, II, III, IV und andere Antibiotika als Nebenbestandteile

2-100% Methanol in Chloroform

Flashsäulenchromatographie (Kieselgel)

1-2% Methanol in Chloroform

2-100% Methanol in Chloroform

Neoviridogrisein I, II, III und IV

Sephadex(R)-LH-20-Chromatographie, Chloroform/Methanol (1:1)

Neoviridogrisein I, II, III und IV (Kristallisation aus Chloroform/Hexan) (60 mg/l)

Grividomycin I, II und III und Griseoviridin (1,5 mg/Liter)

Mitteldruckflüssigkeitschromatographie (Kieselgel)

1 % Methanol in Essigester

Grividomycin I und II

Grividomycin III

Griseoviridin

1. Chromatographie an Kieselgel (30 μ)
2. Elution mit $CHCl_3$ → $CHCl_3$:MeOH (96:4)

Grividomycin I

Grividomycin II

Ungefähre Zusammensetzung der Antibiotika in Prozent

| Neoviridogrisein | | | | Grividomycin | | | Griseoviridin |
|---|---|---|---|---|---|---|---|
| I | II | III | IV | I | II | III | |
| 0,02 | 2 | 0,01 | 95,6 | 0,07 | 0,1 | 0,2 | 2,0 |

Die Homogenität der Grividomycine I, II und III wurde anhand ihrer Dünnschichtchromatogramme auf Kieselgel mit 7,5% Methanol in Chloroform als Laufmittel sowie anhand der Hochdruckflüssigkeitschromatographie auf LiChrosorb (R) RP-18 mit Acetonitril/25 mM Ammoniumacetat/Essigsäure im Verhältnis 150:100:3 als Elutionsmittel überprüft.

BEISPIEL III

Züchtung von Streptomyces sp. Y - 8240155 für die fermentative Herstellung der Antibiotikakomplexes

Die Verfahrensweise des Beispiels II wurde mit den folgenden Unterschieden wiederholt:

Zusammensetzung des Agarmediums

| | |
|---|---|
| Stärke (löslich) | ... 10,0 g |
| $K_2HPO_4$ | ... 1,0 g |
| $MgSO_4 \cdot 7H_2O$ | ... 1,0 g |
| NaCl | ... 1,0 g |
| $(NH_4)_2SO_4$ | ... 2,0 g |
| $CaCO_3$ | ... 2,0 g |
| $FeSO_4 \cdot 7H_2O$ | ... 0,1 mg |
| $MnCl_2 \cdot 4H_2O$ | ... 0,1 mg |
| $ZnSO_4 \cdot 7H_2O$ | ... 0,1 mg |
| Agar | ... 15,0 g |
| Destilliertes Wasser | ... 1 Liter |
| pH | ... 7,2 |

Zusammensetzung des Fermentationsmediums

| | |
|---|---|
| Glucose | ... 20,0 g |
| Sojabohnenmehl | ... 10,0 g |
| $CaCO_3$ | ... 0,2 g |
| $CoCl_2 \cdot 6H_2O$ | ... 0,1 mg |
| Destilliertes Wasser | ... 1 Liter |
| pH | ... 7,0 |

Der Fermenter wurde nach Ablauf von 40 - 44 Stunden abgeerntet. Die Kulturbouillon wurde wie in Beispiel II verarbeitet.

Beispiel IV

Die Verfahrensweise des Beispiels II wurde mit den folgenden Änderungen wiederholt:

Zusammensetzung des Impfkulturmediums

| | | |
|---|---|---|
| Stärke | ... | 20.0 g |
| Sojabohnenmehl | ... | 25.0 g |
| Glucose | ... | 5.0 g |
| $K_2HPO_4$ | ... | 1.0 g |
| $MgSO_4 \cdot 7H_2O$ | ... | 0.5 g |
| Pepton | ... | 1.0 g |
| $CaCl_2$ | ... | 1.0 g |
| NaCl | ... | 3.0 g |
| Destilliertes Wasser | ... | 1 Liter |
| pH | ... | 7.0 |

Zusammensetzung des Fermentationsmediums

| | | |
|---|---|---|
| Dextrin | ... | 20.0 g |
| Sojabohnenmehl | ... | 10.0 g |
| Hefeextrakt | ... | 1.0 g |
| $FeSO_4 \cdot 7H_2O$ | ... | 0.1 g |
| Destilliertes Wasser | ... | 1 Liter |
| pH | ... | 7.0 |

Nach Beendigung der Fermentation (44 - 48 Std.) betrug die Antibiotikakonzentration 40 - 60 mg/l, der pH der Kulturlösung 6.8 und das Mycelvolumen (naß) 8 Vol.-%. Die abgeerntete, den Antibiotikakomplex enthaltende Kulturboullion werde zur Abtrennung des Mycels von der Kulturflüssigkeit zentrifugiert. Diese wurden dann gemäß dem nachstehenden Flußdiagramm aufgearbeitet.

Diagramm II

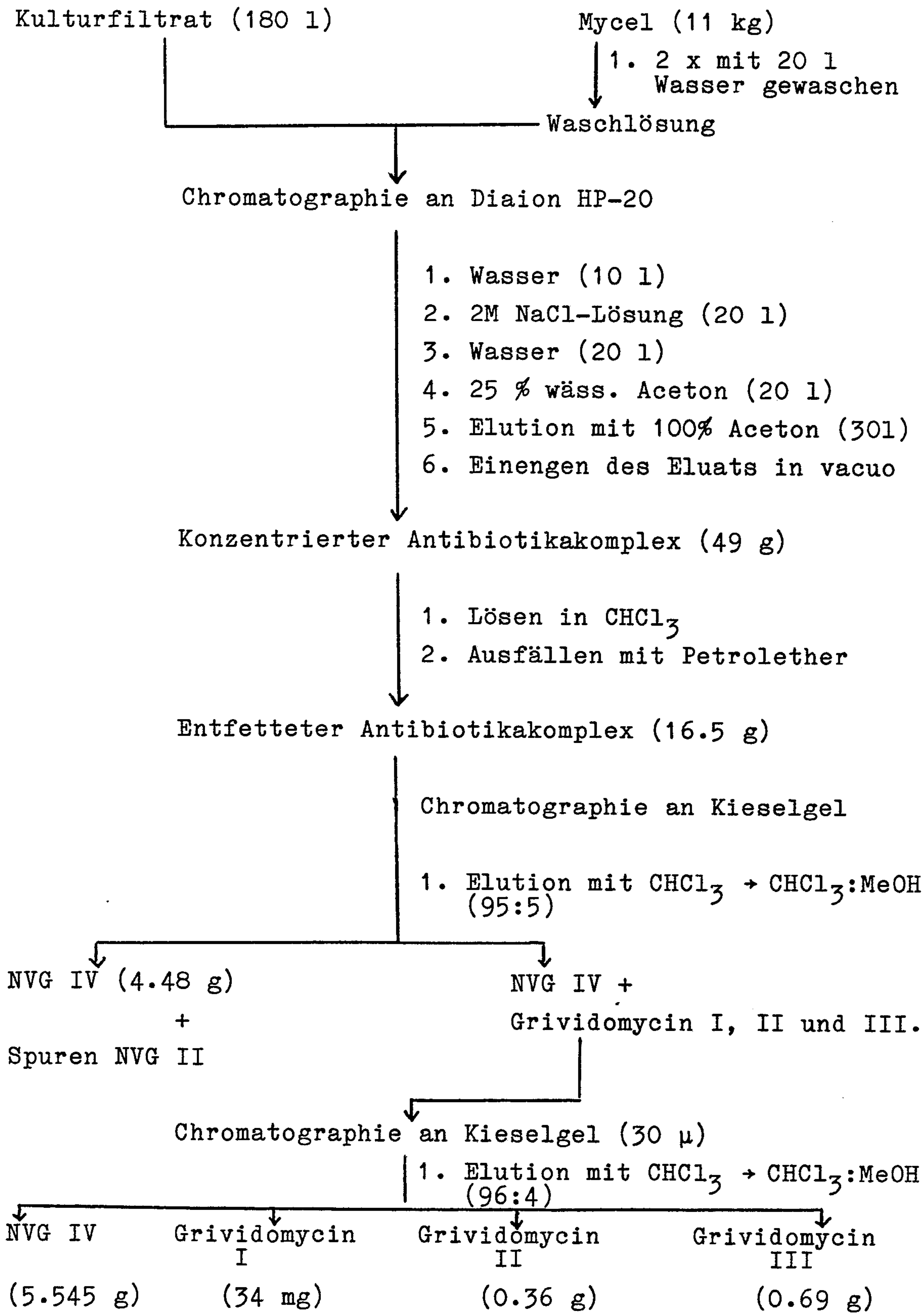
Kulturfiltrat (180 l)
Mycel (11 kg)
1. 2 x mit 20 l Wasser gewaschen
Waschlösung
Chromatographie an Diaion HP-20
1. Wasser (10 l)
2. 2M NaCl-Lösung (20 l)
3. Wasser (20 l)
4. 25 % wäss. Aceton (20 l)
5. Elution mit 100% Aceton (30l)
6. Einengen des Eluats in vacuo
Konzentrierter Antibiotikakomplex (49 g)
1. Lösen in CHCl3
2. Ausfällen mit Petrolether
Entfetteter Antibiotikakomplex (16.5 g)
Chromatographie an Kieselgel
1. Elution mit CHCl3 → CHCl3:MeOH (95:5)
NVG IV (4.48 g)
+
Spuren NVG II
NVG IV +
Grividomycin I, II und III.
Chromatographie an Kieselgel (30 µ)
1. Elution mit CHCl3 → CHCl3:MeOH (96:4)
NVG IV
(5.545 g)
Grividomycin I
(34 mg)
Grividomycin II
(0.36 g)
Grividomycin III
(0.69 g)

Bei diesem Fermentations- und Aufarbeitungsverfahren war die Zusammensetzung des Antibiotikakomplexes wie folgt:

| Neoviridogriseine | | Grividomycine | | |
|---|---|---|---|---|
| II | IV | I | II | III |
| 0.1 % | 90.2 % | 0.3 % | 3.3 % | 6.1 % |

Weder Griseoviridin noch NVG I oder III waren nachweisbar.

Beispiel V

Als ein repräsentatives, nicht limitierendes Beispiel wurde der Antibiotikakomplex zusammengesetzt aus NVG IV und den Grividomycinen I, II, III im Verhältnis von 95:5, in einem Fütterungsversuch mit 180 männlichen Broilerküken verwendet. Die Küken waren 1 Tag alt, hatten ein durchschnittliches Anfangsgewicht von 39,6 g und wurden in 5 Gruppen zu je 36 Küken pro Gruppe aufgeteilt, wie unten angegeben.

Der Fütterungsversuch wurde über 42 Tage durchgeführt.

| Substanz | Konz. ppm | Gew.zun. in g | Gew.zun. in % | Futter-aufn. g | Futter-aufn. % | Futter-verw. % |
|---|---|---|---|---|---|---|
| Kontrolle | - | 1354 | 100 | 2903 | 100 | 100 |
| NVG IV: Grividomycin Mischung | 5 | 1399 | 103,4 | 2817 | 97,0 | 93,9 |
| NVG IV: Grividomycin Mischung | 10 | 1361 | 100,6 | 2759 | 95,0 | 94,5 |
| NVG IV: Grividomycin | 20 | 1410 | 104,1 | 2853 | 98,0 | 94,4 |

Die Ergebnisse des Versuchs zeigen eine bessere Futterverwertung und eine höhere Körpergewichtszunahme im Vergleich zur Kontrollgruppe, welche keinen Zusatz im Futter erhalten hatte.

Beispiel VI

Für diesen Fütterungsversuch wurde sprühgetrocknete Kulturlösung benutzt, welche zu 0,2 % den Antibiotikakomplex enthielt (d.h. 2 g/kg). Die Zusammensetzung des Komplexes war NVG IV : Grividomycin : NVG II im Verhältnis von 96 : 3 : 1.

Der Fütterungsversuch wurde ähnlich durchgeführt wie im Beispiel V bereits beschrieben, mit den folgenden Abweichungen:
160 männliche Broilerküken, 1 Tag alt, mit einem Anfangsgewicht von 37,2 g wurden in 5 Gruppen mit je 32 Tieren pro Gruppe aufgeteilt, wie unten dargestellt.

| Substanz | Konz. ppm | Gew.zun. in g | Gew.zun. in % | Futter-aufn. g | Futter-aufn. % | Futter-verw. % |
|---|---|---|---|---|---|---|
| Kontrolle | - | 1355 | 100 | 2558 | 100 | 100 |
| NVG IV: Grividomycin Mischung | 5 | 1448 | 106,9 | 2734 | 106,9 | 100 |
| NVG IV: Grividomycin Mischung | 10 | 1441 | 106,4 | 2683 | 104,9 | 98,6 |
| NVG IV: Grividomycin Mischung | 20 | 1452 | 107,2 | 2724 | 106,5 | 99,4 |

Patentansprüche:

1. Verfahren zur Herstellung eines Antibiotikakomplexes enthaltend Grividomycin I und II der Formel Ia

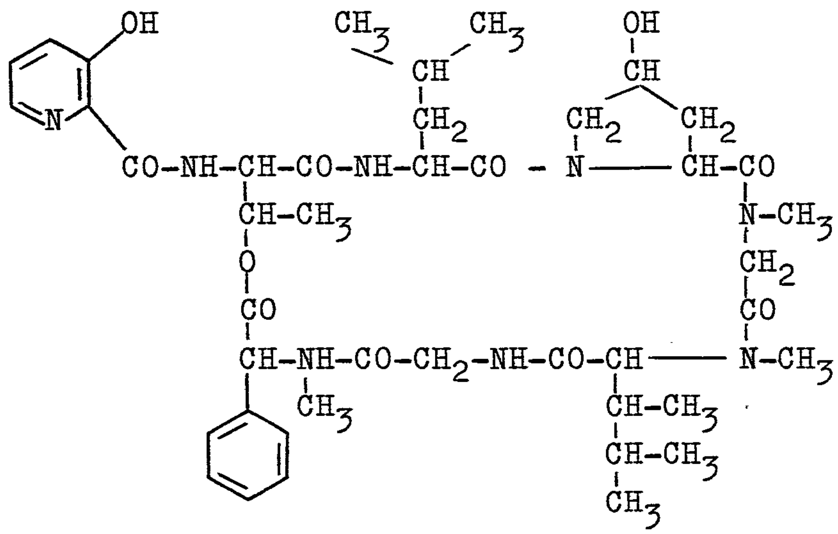

Ia

Grividomycin III der Formel Ib

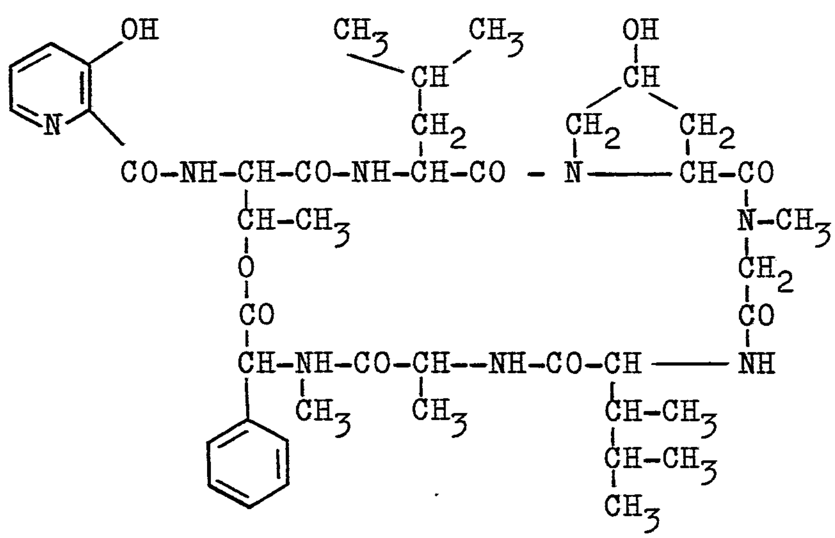

Ib

Neoviridogrisein IV, Neoviridogrisein II und gegebenenfalls Griseoviridin, Neoviridogrisein I und III, dadurch gekennzeichnet, daß man den Mikroorganismus

Streptomyces sp. Y - 8240155 (DSM 3640) oder eine seiner Mutanten oder Varianten bei einem pH von 6,5 bis 8,5 und einer Temperatur zwischen 20 und 40°C unter aeroben Bedingungen in einem Kohlenstoff- und Stickstoffquellen, anorganische Nährsalze und Spurenelemente enthaltenden Nährmedium bis zum Erreichen einer wesentlichen antibiotischen Wirksamkeit kultiviert und das Fermentationsprodukt aus dem Kulturmedium in an sich bekannter Weise isoliert.

2. Verfahren gemäß Anspruch 1, dadurch gekennzeichnet, daß man den Mikroorganismus bei pH 6,5 - 7,0 und einer Temperatur von 25 bis 35°C etwa 40 bis 50 Stunden kultiviert.

3. Verfahren zur Trennung des nach Anspruch 1 erhaltenen Antibiotikakomplexes, dadurch gekennzeichnet, daß man a) das Kulturfiltrat bei pH 4,5 bis 7,5 mit einem nicht wassermischbaren organischen Lösungsmittel und b) das Mycel zunächst mit Aceton und den Acetonextract bei pH 4,5 bis 7,5 mit einem nicht wassermischbaren organischen Lösungsmittel extrahiert und die vereinigten Extrakte trennt.

4. Verfahren zur Herstellung von Grividomycin I der Formel Ia, dadurch gekennzeichnet, daß man den Mikroorganismus Streptomyces sp. Y - 8240155 (DSM 3640) oder eine seiner Mutanten oder Varianten bei einem pH von 6,5 bis 8,5 und einer Temperatur zwischen 20 und 40°C unter aeroben Bedingungen in einem Kohlenstoff- und Stickstoffquellen, anorganische Nährsalze und Spurenelemente enthaltenden Nährmedium bis zum Erreichen einer wesentlichen antibiotischen Wirksamkeit kultiviert, das Fermentationsprodukt aus dem Kulturmedium abtrennt und daraus Grividomycin I isoliert.

5. Verfahren zur Herstellung von Grividomycin II der Formel Ia, dadurch gekennzeichnet, daß man den Mikroorganismus Streptomyces sp. Y - 8240155 (DSM 3640) oder eine seiner Mutanten oder Varianten bei einem pH von 6,5 bis 8,5 und einer Temperatur zwischen 20 und 40°C unter aeroben Bedingungen in einem Kohlenstoff- und Stickstoffquellen, anorganische Nährsalze und Spurenelemente enthaltenden Nährmedium bis zum Erreichen einer wesentlichen antibiotischen Wirksamkeit kultiviert, das Fermentationsprodukt aus dem Kulturmedium abtrennt und daraus Grividomycin II isoliert.

6. Verfahren zur Herstellung von Grividomycin III der Formel Ib, dadurch gekennzeichnet, daß man den Mikroorganismus Streptomyces sp. Y - 8240155 (DSM 3640) oder eine seiner Mutanten oder Varianten bei einem pH von 6,5 bis 8,5 und einer Temperatur zwischen 20 und 40°C unter aeroben Bedingungen in einem Kohlenstoff- und Stickstoffquellen, anorganische Nährsalze und Spurenelemente enthaltenden Nährmedium bis zum Erreichen einer wesentlichen antibiotischen Wirksamkeit kultiviert, das Fermentationsprodukt aus dem Kulturmedium abtrennt und daraus Grividomycin III isoliert.

7. Verbindung der Formel Ia.

8. Verbindung der Formel Ib.

9. Antibiotikakomplex bestehend aus Grividomycin I, II und III, Neoviridogrisein IV und II sowie gegebenenfalls Griseoviridin, Neoviridogrisein I und III, hergestellt durch Kultivierung des Mikroorganismus Streptomyces sp. Y-8240155 (DMS 3640) und Isolierung des Komplexes gemäß dem Verfahren des Anspruchs 1.

10. Antibiotikakomplex gemäß Anspruch 9, dadurch gekennzeichnet, daß er zu mindestens 90 % aus Neoviridogrisein IV besteht.

11. Futterzusatz, gekennzeichnet durch einen Gehalt an einem durch Züchtung von Streptomyces sp. Y-8240155 (DMS 3640) bzw. seiner Varianten und Mutanten erhaltenen Antiobiotikakomplex.

12. Futterzusatz gemäß Anspruch 11, gekennzeichnet durch einen Gehalt an dem bei der Züchtung von Streptomyces sp. Y-8240155 (DMS 3640) bzw. seiner Varianten und Mutanten erhaltenen Trockenmycel.

13. Futterzusatz gemäß Anspruch 11, gekennzeichnet durch einen Gehalt an dem durch Züchtung von Streptomyces sp. Y-8240155 (DMS 3640) bzw. seiner Varianten und Mutanten und Isolierung aus Mycel und Kulturfiltrat erhaltenen Antibiotikakomplex.

14. Futterzusatz, gekennzeichnet durch einen Gehalt an Grividomycin I, II und/oder III.

15. Futterzusatz gemäß Anspruch 14, gekennzeichnet durch einen Gehalt an Neoviridogrisein IV neben Grividomycin I, II und III.

16. Futterzusatz, dadurch gekennzeichnet, daß er Grividomycin I, II und III, Neoviridogrisein II und IV sowie gegebenenfalls Griseoviridin und Neoviridogrisein I und III enthält.

17. Futterzusatz gemäß Anspruch 16, dadurch gekennzeichnet, daß der Anteil an Neoviridogrisein IV mindestens 90 % beträgt.

18. Arzneimittel zur Behandlung von bakteriellen Infektionen, enthaltend als Wirkstoff Grividomycin I, II oder III.

HOE 86/F 038
0232893

19. Verwendung eines Antibiotikakomplexes gemäß Anspruch 9 als Futterzusatz.

20. Verwendung des bei der Züchtung von Streptomyces sp. bzw. seiner Varianten und Mutanten erhaltenen Trockenmycels als Futterzusatz.

21. Verwendung von Neoviridogrisein IV (allein oder im Gemisch mit Grividomycin I, II und/oder III) als Futterzusatz.

22. Verfahren zur Wachstumsförderung bei Tieren, dadurch gekennzeichnet, daß man den Tieren eine wirksame Menge eines Antibiotikakomplexes gemäß Anspruch 9 oral verabreicht.

23. Verfahren gemäß Anspruch 22, dadurch gekennzeichnet, daß man den Antibiotikakomplex dem Tierfutter oder einer Beifüttermischung zumischt.

24. Verfahren gemäß Anspruch 22, dadurch gekennzeichnet, daß die wirksame Menge einer Konzentration von 2 - 100 ppm, vorzugsweise 2 - 50 ppm, insbesondere 5 - 20 ppm, bezogen auf Alleinfutter, entspricht.

25. Verfahren gemäß Anspruch 22, dadurch gekennzeichnet, daß man den Antibiotikakomplex in Form eines Trockenmycels einsetzt.

26. Streptomyces sp. Y-8240155 (DMS 3640)

Patentansprüche Griechenland und Österreich:

1. Verfahren zur Herstellung eines Antibiotikakomplexes enthaltend Grividomycin I und II der Formel Ia

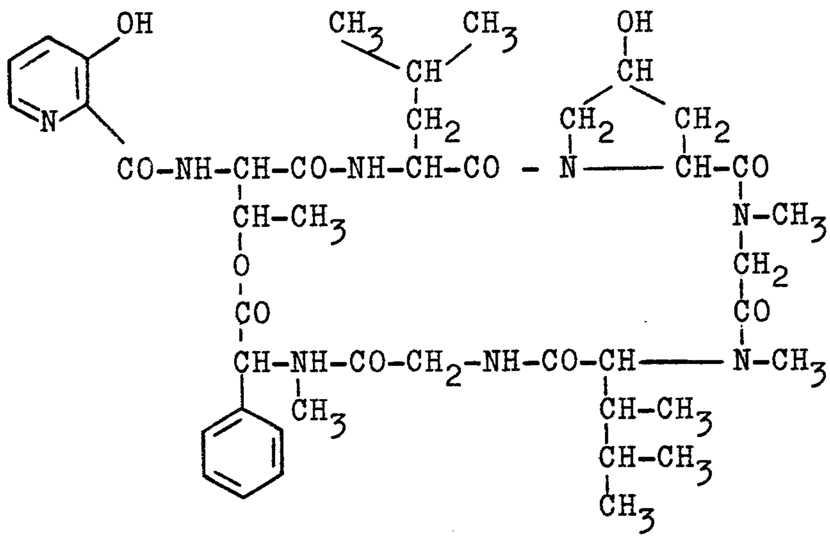

Ia

Grividomycin III der Formel Ib

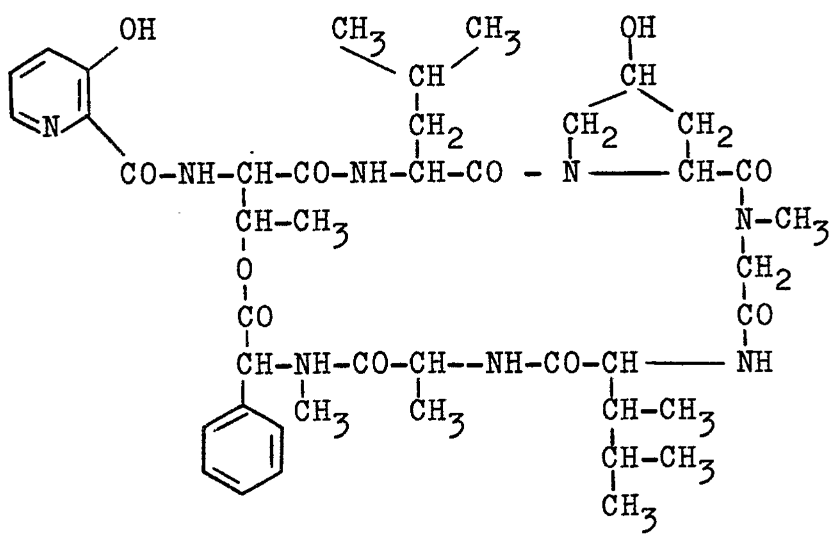

Ib

Neoviridogrisein IV, Neoviridogrisein II und gegebenenfalls Griseoviridin, Neoviridogrisein I und III, dadurch gekennzeichnet, daß man den Mikroorganismus

Streptomyces sp. Y - 8240155 (DSM 3640) oder eine seiner Mutanten oder Varianten bei einem pH von 6,5 bis 8,5 und einer Temperatur zwischen 20 und 40°C unter aeroben Bedingungen in einem Kohlenstoff- und Stickstoffquellen, anorganische Nährsalze und Spurenelemente enthaltenden Nährmedium bis zum Erreichen einer wesentlichen antibiotischen Wirksamkeit kultiviert und das Fermentationsprodukt aus dem Kulturmedium in an sich bekannter Weise isoliert.

2. Verfahren gemäß Anspruch 1, dadurch gekennzeichnet, daß man den Mikroorganismus bei pH 6,5 - 7,0 und einer Temperatur von 25 bis 35°C etwa 40 bis 50 Stunden kultiviert.

3. Verfahren zur Trennung des nach Anspruch 1 erhaltenen Antibiotikakomplexes, dadurch gekennzeichnet, daß man a) das Kulturfiltrat bei pH 4,5 bis 7,5 mit einem nicht wassermischbaren organischen Lösungsmittel und b) das Mycel zunächst mit Aceton und den Acetonextract bei pH 4,5 bis 7,5 mit einem nicht wassermischbaren organischen Lösungsmittel extrahiert und die vereinigten Extrakte trennt.

4. Verfahren zur Herstellung von Grividomycin I der Formel Ia, dadurch gekennzeichnet, daß man den Mikroorganismus Streptomyces sp. Y - 8240155 (DSM 3640) oder eine seiner Mutanten oder Varianten bei einem pH von 6,5 bis 8,5 und einer Temperatur zwischen 20 und 40°C unter aeroben Bedingungen in einem Kohlenstoff- und Stickstoffquellen, anorganische Nährsalze und Spurenelemente enthaltenden Nährmedium bis zum Erreichen einer wesentlichen antibiotischen Wirksamkeit kultiviert, das Fermentationsprodukt aus dem Kulturmedium abtrennt und daraus Grividomycin I isoliert.

5. Verfahren zur Herstellung von Grividomycin II der Formel Ia, dadurch gekennzeichnet, daß man den Mikroorganismus Streptomyces sp. Y - 8240155 (DSM 3640) oder eine seiner Mutanten oder Varianten bei einem pH von 6,5 bis 8,5 und einer Temperatur zwischen 20 und 40°C unter aeroben Bedingungen in einem Kohlenstoff- und Stickstoffquellen, anorganische Nährsalze und Spurenelemente enthaltenden Nährmedium bis zum Erreichen einer wesentlichen antibiotischen Wirksamkeit kultiviert, das Fermentationsprodukt aus dem Kulturmedium abtrennt und daraus Grividomycin II isoliert.

6. Verfahren zur Herstellung von Grividomycin III der Formel Ib, dadurch gekennzeichnet, daß man den Mikroorganismus Streptomyces sp. Y - 8240155 (DSM 3640) oder eine seiner Mutanten oder Varianten bei einem pH von 6,5 bis 8,5 und einer Temperatur zwischen 20 und 40°C unter aeroben Bedingungen in einem Kohlenstoff- und Stickstoffquellen, anorganische Nährsalze und Spurenelemente enthaltenden Nährmedium bis zum Erreichen einer wesentlichen antibiotischen Wirksamkeit kultiviert, das Fermentationsprodukt aus dem Kulturmedium abtrennt und daraus Grividomycin III isoliert.

7. Antibiotikakomplex bestehend aus Grividomycin I, II und III, Neoviridogrisein IV und II sowie gegebenenfalls Griseoviridin, Neoviridogrisein I und III, hergestellt durch Kultivierung des Mikroorganismus Streptomyces sp. Y-8240155 (DMS 3640) und Isolierung des Komplexes gemäß dem Verfahren des Anspruchs 1.

8. Antibiotikakomplex gemäß Anspruch 7, dadurch gekennzeichnet, daß er zu mindestens 90 % aus Neoviridogrisein IV besteht.

9. Futterzusatz, gekennzeichnet durch einen Gehalt an einem durch Züchtung von Streptomyces sp. Y-8240155 (DMS 3640) bzw. seiner Varianten und Mutanten erhaltenen Antiobiotikakomplex.

10. Futterzusatz gemäß Anspruch 9, gekennzeichnet durch einen Gehalt an dem bei der Züchtung von Streptomyces sp. Y-8240155 (DMS 3640) bzw. seiner Varianten und Mutanten erhaltenen Trockenmycel.

11. Futterzusatz gemäß Anspruch 9, gekennzeichnet durch einen Gehalt an dem durch Züchtung von Streptomyces sp. Y-8240155 (DMS 3640) bzw. seiner Varianten und Mutanten und Isolierung aus Mycel und Kulturfiltrat erhaltenen Antibiotikakomplex.

12. Futterzusatz, gekennzeichnet durch einen Gehalt an Grividomycin I, II und/oder III.

13. Futterzusatz gemäß Anspruch 12, gekennzeichnet durch einen Gehalt an Neoviridogrisein IV neben Grividomycin I, II und III.

14. Futterzusatz, dadurch gekennzeichnet, daß er Grividomycin I, II und III, Neoviridogrisein II und IV sowie gegebenenfalls Griseoviridin und Neoviridogrisein I und III enthält.

15. Futterzusatz gemäß Anspruch 13, dadurch gekennzeichnet, daß der Anteil an Neoviridogrisein IV mindestens 90 % beträgt.

16. Arzneimittel zur Behandlung von bakteriellen Infektionen, enthaltend als Wirkstoff Grividomycin I, II oder III.

17. Verwendung eines Antibiotikakomplexes gemäß Anspruch 7 als Futterzusatz.

18. Verwendung des bei der Züchtung von Streptomyces sp. bzw. seiner Varianten und Mutanten erhaltenen Trockenmycels als Futterzusatz.

19. Verwendung von Neoviridogrisein IV (allein oder im Gemisch mit Grividomycin I, II und/oder III) als Futterzusatz.

20. Verfahren zur Wachstumsförderung bei Tieren, dadurch gekennzeichnet, daß man den Tieren eine wirksame Menge eines Antibiotikakomplexes gemäß Anspruch 10 oral verabreicht.

21. Verfahren gemäß Anspruch 20, dadurch gekennzeichnet, daß man den Antibiotikakomplex dem Tierfutter oder einer Beifüttermischung zumischt.

22. Verfahren gemäß Anspruch 20, dadurch gekennzeichnet, daß die wirksame Menge einer Konzentration von 2 - 100 ppm, vorzugsweise 2 - 50 ppm, insbesondere 5 - 20 ppm, bezogen auf Alleinfutter, entspricht.

23. Verfahren gemäß Anspruch 20, dadurch gekennzeichnet, daß man den Antibiotikakomplex in Form eines Trockenmycels einsetzt.

Patentansprüche Spanien:

1. Verfahren zur Herstellung eines Antibiotikakomplexes enthaltend Grividomycin I und II der Formel Ia

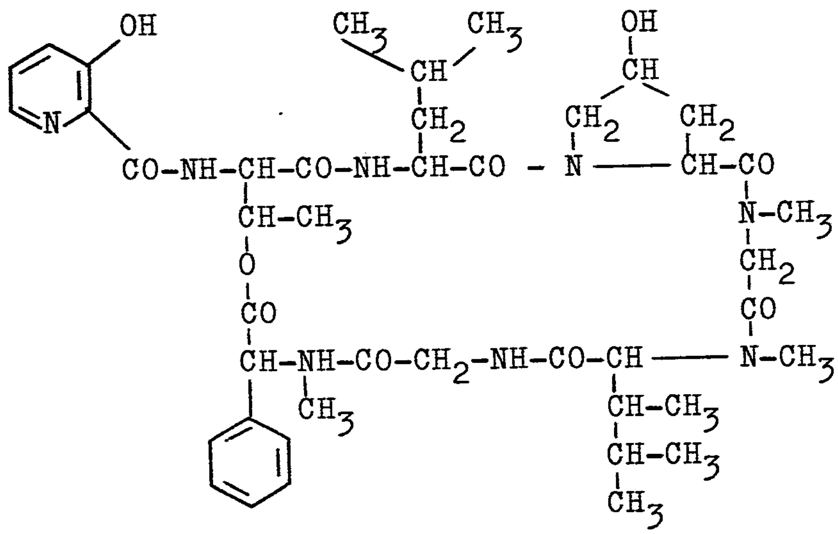

Ia

Grividomycin III der Formel Ib

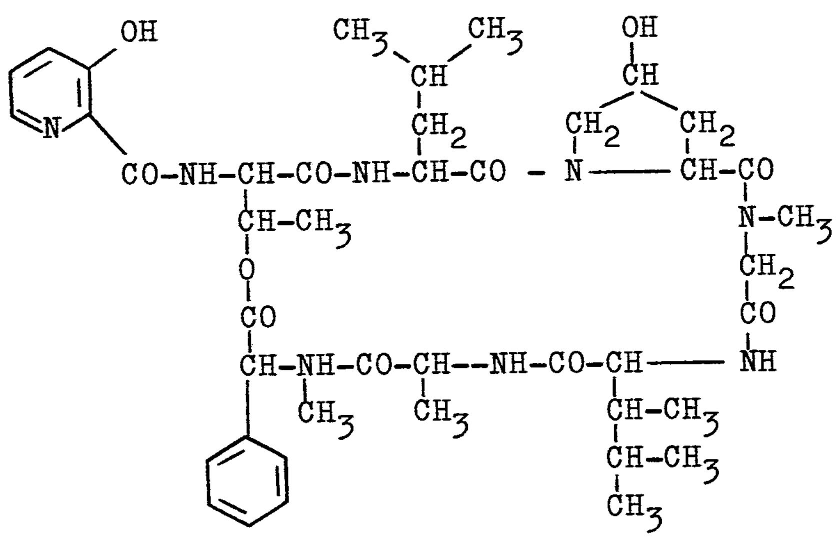

Ib

Neoviridogrisein IV, Neoviridogrisein II und gegebenenfalls Griseoviridin, Neoviridogrisein I und III, dadurch gekennzeichnet, daß man den Mikroorganismus

Streptomyces sp. Y - 8240155 (DSM 3640) oder eine seiner Mutanten oder Varianten bei einem pH von 6,5 bis 8,5 und einer Temperatur zwischen 20 und 40°C unter aeroben Bedingungen in einem Kohlenstoff- und Stickstoffquellen, anorganische Nährsalze und Spurenelemente enthaltenden Nährmedium bis zum Erreichen einer wesentlichen antibiotischen Wirksamkeit kultiviert und das Fermentationsprodukt aus dem Kulturmedium in an sich bekannter Weise isoliert.

2. Verfahren gemäß Anspruch 1, dadurch gekennzeichnet, daß man den Mikroorganismus bei pH 6,5 - 7,0 und einer Temperatur von 25 bis 35°C etwa 40 bis 50 Stunden kultiviert.

3. Verfahren zur Trennung des nach Anspruch 1 erhaltenen Antibiotikakomplexes, dadurch gekennzeichnet, daß man a) das Kulturfiltrat bei pH 4,5 bis 7,5 mit einem nicht wassermischbaren organischen Lösungsmittel und b) das Mycel zunächst mit Aceton und den Acetonextract bei pH 4,5 bis 7,5 mit einem nicht wassermischbaren organischen Lösungsmittel extrahiert und die vereinigten Extrakte trennt.

4. Verfahren zur Herstellung von Grividomycin I der Formel Ia, dadurch gekennzeichnet, daß man den Mikroorganismus Streptomyces sp. Y - 8240155 (DSM 3640) oder eine seiner Mutanten oder Varianten bei einem pH von 6,5 bis 8,5 und einer Temperatur zwischen 20 und 40°C unter aeroben Bedingungen in einem Kohlenstoff- und Stickstoffquellen, anorganische Nährsalze und Spurenelemente enthaltenden Nährmedium bis zum Erreichen einer wesentlichen antibiotischen Wirksamkeit kultiviert, das Fermentationsprodukt aus dem Kulturmedium abtrennt und daraus Grividomycin I isoliert.

5. Verfahren zur Herstellung von Grividomycin II der Formel Ia, dadurch gekennzeichnet, daß man den Mikroorganismus Streptomyces sp. Y - 8240155 (DSM 3640) oder eine seiner Mutanten oder Varianten bei einem pH von 6,5 bis 8,5 und einer Temperatur zwischen 20 und 40°C unter aeroben Bedingungen in einem Kohlenstoff- und Stickstoffquellen, anorganische Nährsalze und Spurenelemente enthaltenden Nährmedium bis zum Erreichen einer wesentlichen antibiotischen Wirksamkeit kultiviert, das Fermentationsprodukt aus dem Kulturmedium abtrennt und daraus Grividomycin II isoliert.

6. Verfahren zur Herstellung von Grividomycin III der Formel Ib, dadurch gekennzeichnet, daß man den Mikroorganismus Streptomyces sp. Y - 8240155 (DSM 3640) oder eine seiner Mutanten oder Varianten bei einem pH von 6,5 bis 8,5 und einer Temperatur zwischen 20 und 40°C unter aeroben Bedingungen in einem Kohlenstoff- und Stickstoffquellen, anorganische Nährsalze und Spurenelemente enthaltenden Nährmedium bis zum Erreichen einer wesentlichen antibiotischen Wirksamkeit kultiviert, das Fermentationsprodukt aus dem Kulturmedium abtrennt und daraus Grividomycin III isoliert.

FIG.1

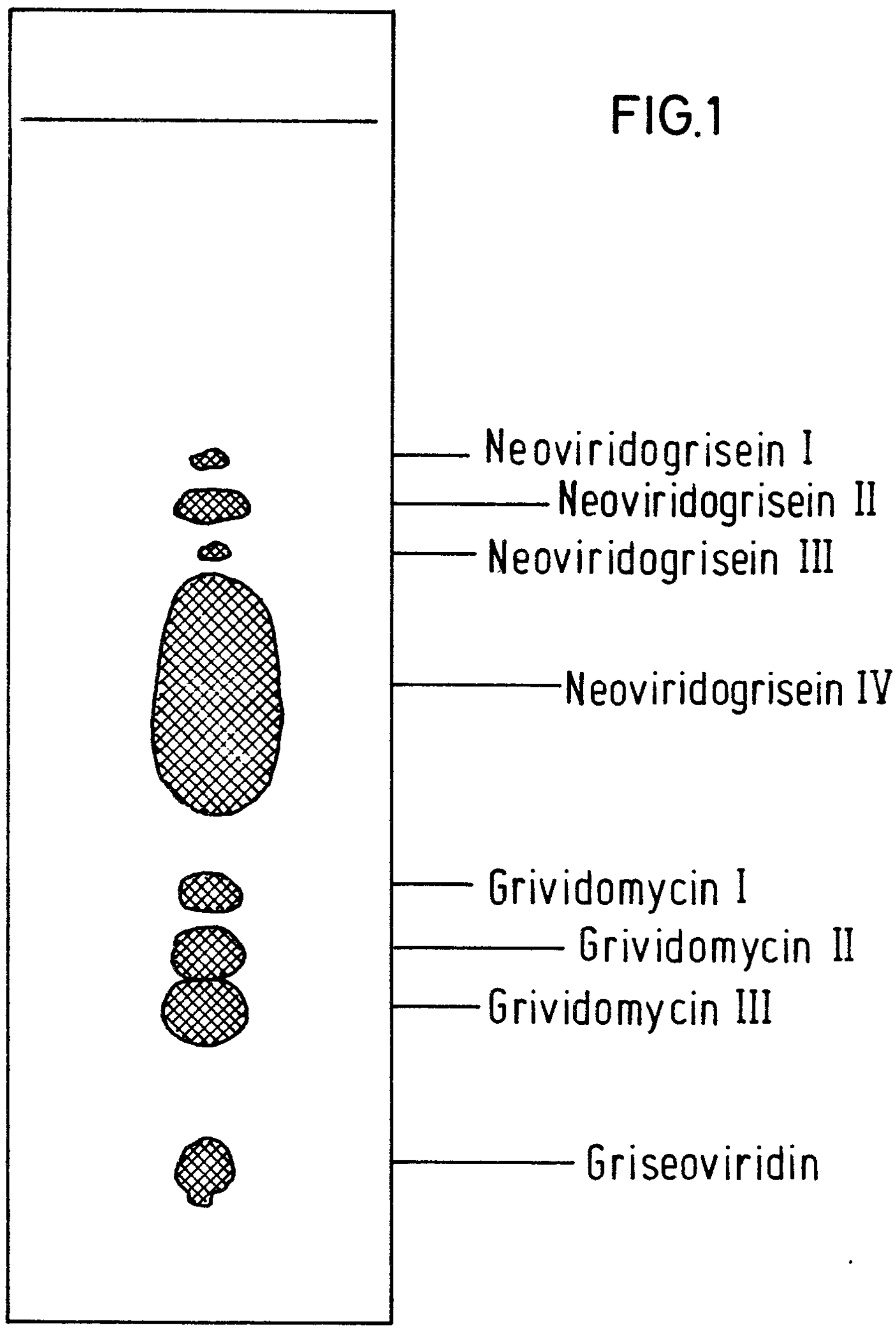
Neoviridogrisein I
Neoviridogrisein II
Neoviridogrisein III
Neoviridogrisein IV
Grividomycin I
Grividomycin II
Grividomycin III
Griseoviridin

0232893

2/7

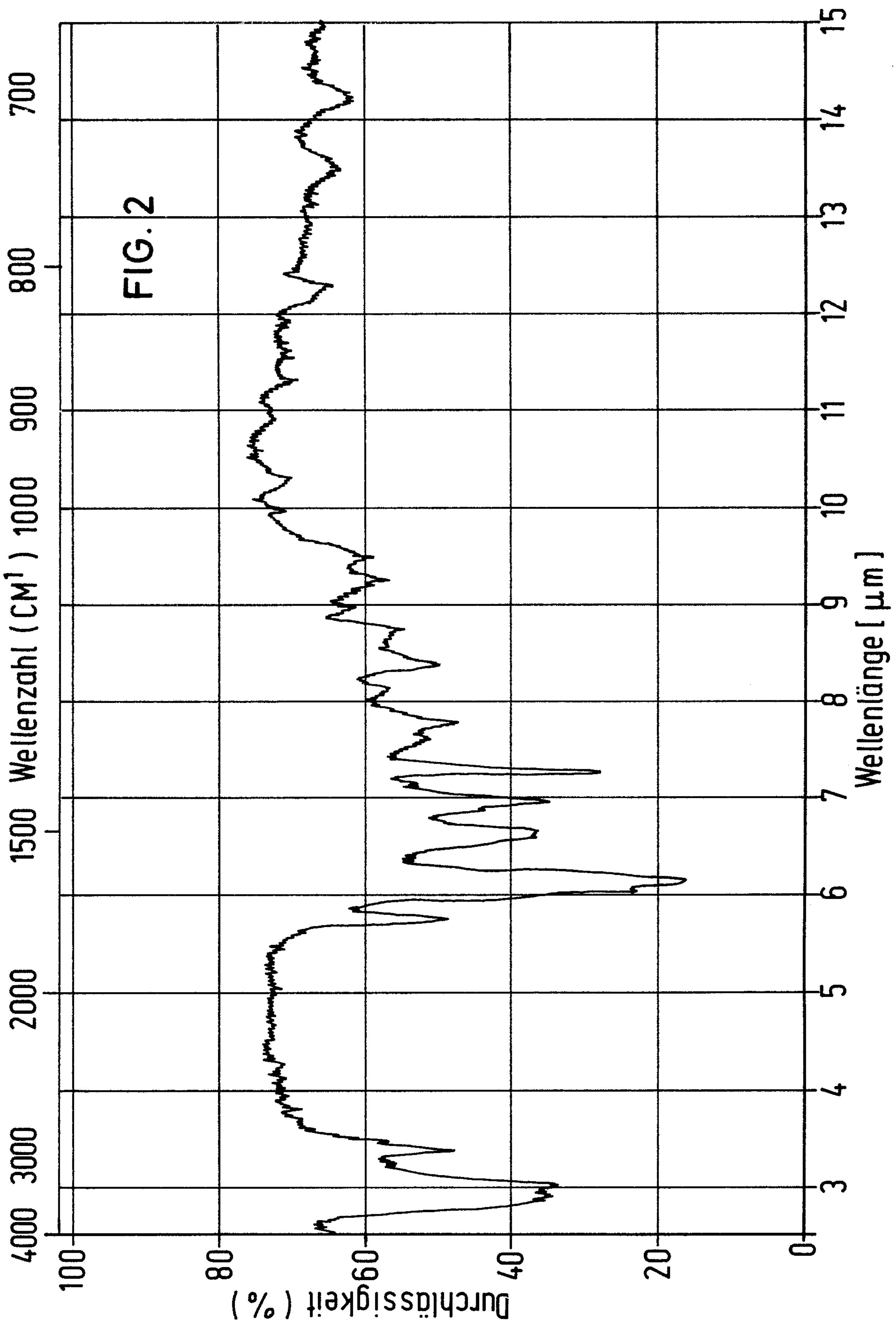


FIG. 2

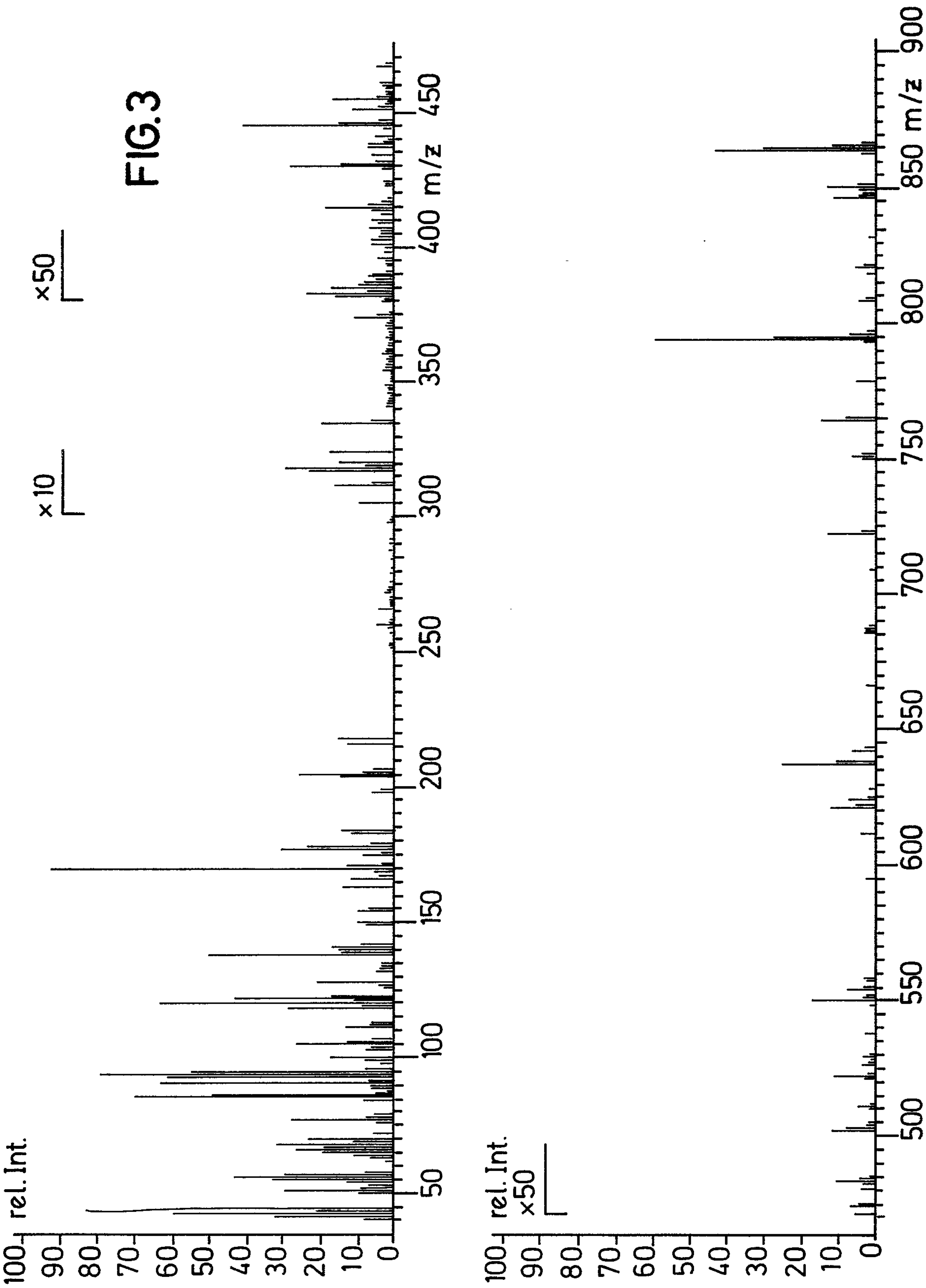


FIG.3

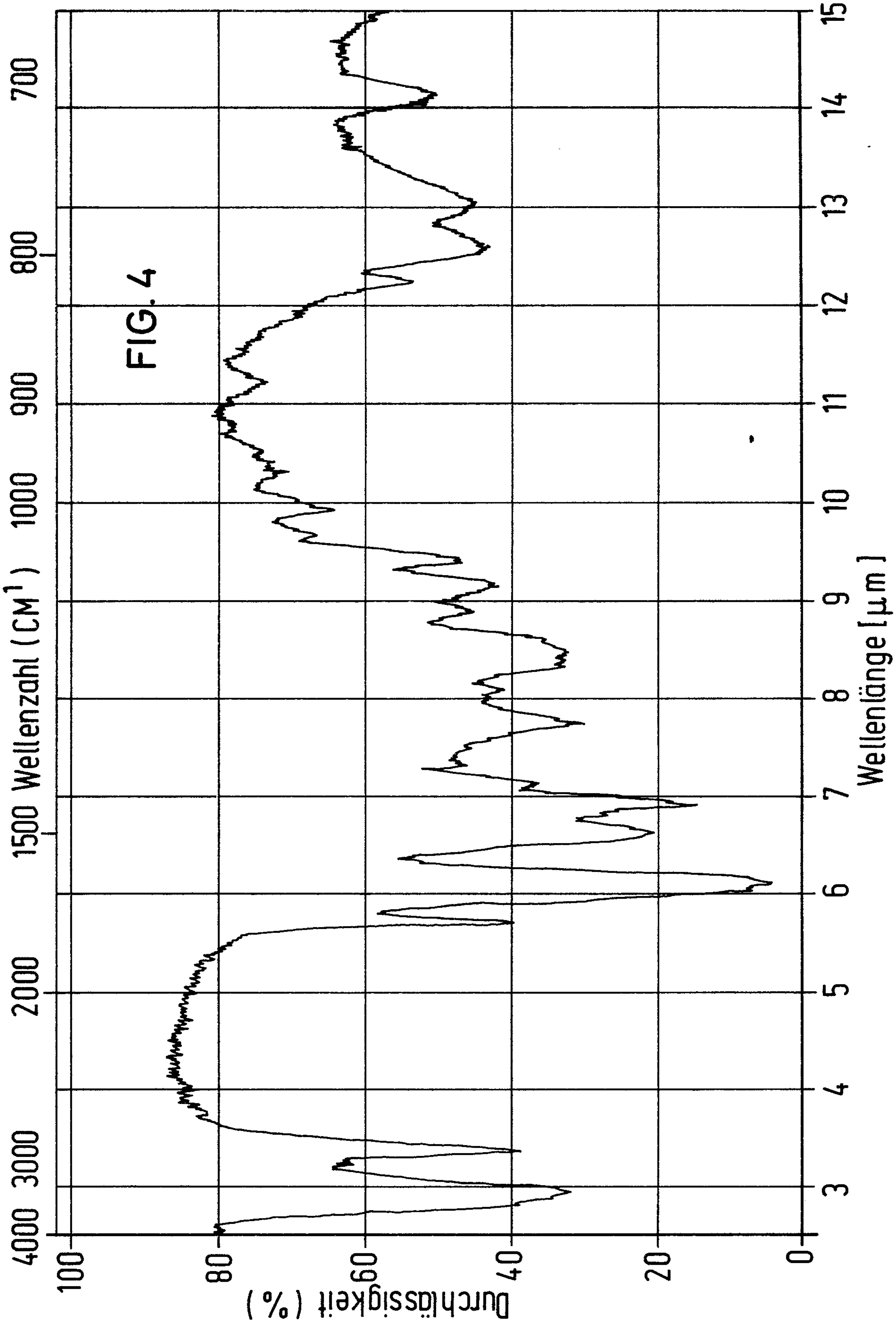
4000 3000 2000 1500 Wellenzahl ( CM$^{-1}$ ) 1000 900 800 700
3 4 5 6 7 8 9 10 11 12 13 14 15
Wellenlänge [ µm ]
100 80 60 40 20 0
Durchlässigkeit ( % )

FIG. 4

FIG. 5

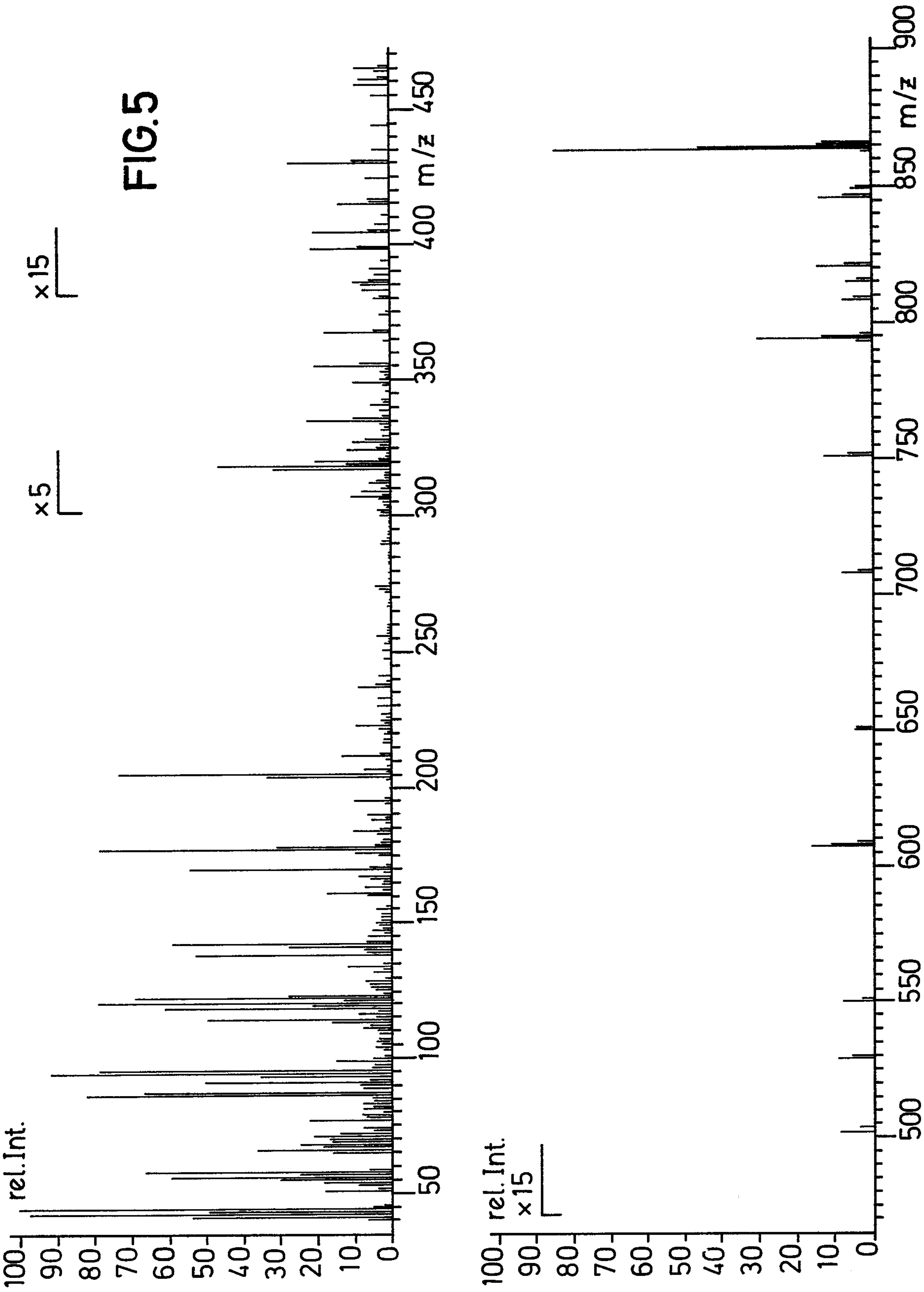
rel. Int.
100 90 80 70 60 50 40 30 20 10 0
×5
×15
50 100 150 200 250 300 350 400 450 m/z
rel. Int.
×15
100 90 80 70 60 50 40 30 20 10 0
500 550 600 650 700 750 800 850 900 m/z

FIG. 6

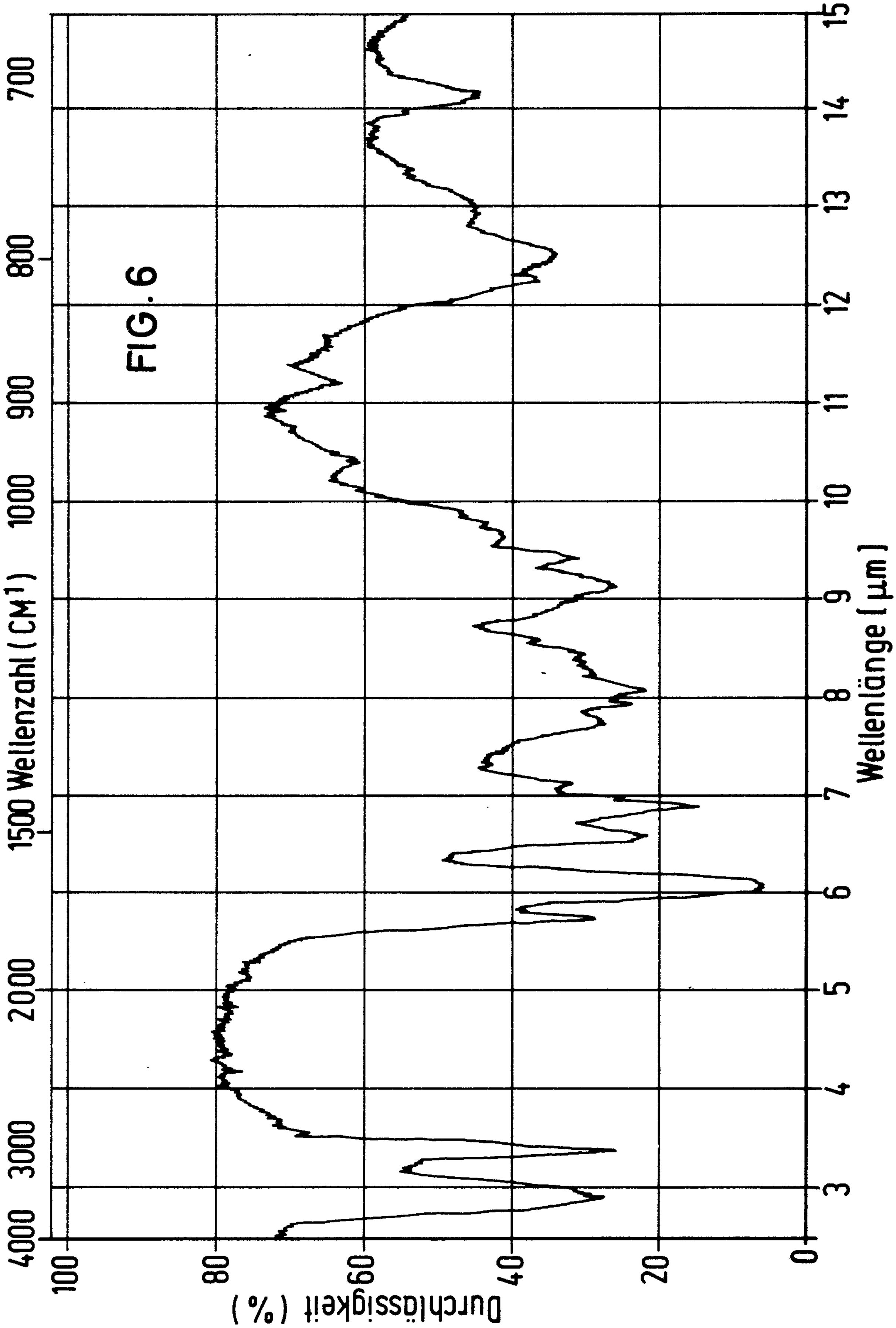
Wellenzahl (CM⁻¹)
4000
3000
2000
1500
1000
900
800
700
Durchlässigkeit (%)
100
80
60
40
20
0
Wellenlänge [µm]
3
4
5
6
7
8
9
10
11
12
13
14
15

FIG.7